(19) Europäisches Patentamt
European Patent Office
Office européen des brevets

(11) EP 2 036 494 A2

(12) EUROPEAN PATENT APPLICATION

(43) Date of publication:
18.03.2009 Bulletin 2009/12

(51) Int Cl.:
A61B 5/06 (2006.01)          A61B 8/12 (2006.01)
A61B 1/05 (2006.01)          A61B 6/03 (2006.01)
A61B 5/055 (2006.01)

(21) Application number: 08007928.8

(22) Date of filing: 24.04.2008

(84) Designated Contracting States:
AT BE BG CH CY CZ DE DK EE ES FI FR GB GR
HR HU IE IS IT LI LT LU LV MC MT NL NO PL PT
RO SE SI SK TR
Designated Extension States:
AL BA MK RS

(30) Priority: 07.05.2007 JP 2007122649
17.05.2007 JP 2007132008

(71) Applicant: OLYMPUS MEDICAL SYSTEMS CORP.
Shibuya-ku
Tokyo 151-0072 (JP)

(72) Inventors:
• Komuro, Masahiko
  Shibuya-ku
  Tokyo 151-0072 (JP)
• Kawashima, Tomonao
  Shibuya-ku
  Tokyo 151-0072 (JP)
• Ikuma, Soichi
  Shibuya-ku
  Tokyo 151-0072 (JP)

(74) Representative: von Hellfeld, Axel
Wuesthoff & Wuesthoff
Patent- und Rechtsanwälte
Schweigerstrasse 2
81541 München (DE)

(54) **Medical guiding system**

(57) A medical guiding system of the invention includes: a detection portion for detecting at least one of position and orientation of a medical instrument; a storage portion for storing multiple reference image data obtained from a human body in multiple states and including anatomical positional information of at least one of viscus and organ; a state selection portion for selecting, among the stored reference image data, the reference image data obtained in a state coincident with or approximate to a state of the subject in using the medical instrument; and a guide image creation portion for creating a guide image showing at least one of an anatomical position, shape, and orientation of the medical instrument with respect to the subject, based on at least one of the position and the orientation of the medical instrument detected by the detection portion and reference image data stored in the state selection portion.

FIG.1

**Description**

BACKGROUND OF THE INVENTION

1. Field of the Invention

The present invention relates to a medical guiding system for creating a guide image showing at least one of an anatomical position, shape, and orientation of a medical instrument with respect to a subject.

2. Description of Related Art

There has been conventionally well-known a medical instrument as typified by an endoscope, an ultrasound endoscope, a small-diameter ultrasound probe or the like, which is introduced into a living body such as gastrointestinal tract, biliopancreatic duct, or blood vessels to be used for diagnosis, treatment, operation and the like. The endoscope includes a bronchoscope, a gastrointestinal endoscope, a laparoscope, and the like.

When performing inspection or operation using the medical instrument to be introduced in a living body, an operator performs diagnosis and surgery assuming anatomical position under current observation, while previously taking into account known anatomical positional relationships of the organs or the tissues in a living body.

In order to assist such diagnosis and surgery using such a medical instrument, there have been proposed techniques for displaying a guide image showing an anatomical position of the medical instrument in a living body, through synthesis based on a CT image or an MRI image obtained in advance.

For example, Japanese Patent Application Laid-Open No. 2005-312770 describes an ultrasound diagnostic apparatus that detects a distal end position of an ultrasound endoscope, constructs a guide image corresponding to an anatomical position of the ultrasound endoscope based on anatomical image data, and displays the constructed image.

Furthermore, Japanese Patent Application Laid-Open No. 2006-149481, International Application No. WO 2006/057296, and Japanese Patent Application Laid-Open No. 2007-37790 each disclose an ultrasound diagnostic apparatus which is provided with three-dimensional guide image creation means and allows easy confirmation of an observation position based on an ultrasound tomographic image by displaying a stereoscopic three-dimensional guide image.

In addition, Japanese Patent Application Laid-Open No. 2002-263101 discloses an ultrasound diagnostic apparatus including: an ultrasound probe to transmit and receive ultrasound to and from a tissue in a living body to output a received signal; ultrasound image creation means for creating an ultrasound image of the tissue based on the received signal; image database in which a plurality of illustration images typically showing inside of a living body are stored; image selection means for selecting an illustration image corresponding to the tissue from the image database; and display means for displaying the selected illustration image together with the ultrasound image.

Incidentally, when introducing a medical instrument into a living body to perform diagnosis, treatment, or operation, it is usual for an operator to change a direction and posture of a living body, or to move, deform the medical instrument or change the direction of the medical instrument in the living body, depending on a region to be inspected.

Therefore, the body position change of the living body or introduction of the medical instrument into the living body sometimes causes a movement or deformation of the organs and the tissues in the living body.

Accordingly, difference occurs between the positions or the shapes of the organs and the tissues in the CT image or the MRI image obtained beforehand and the positions or the shapes of the organs and the tissues at the time of performing diagnosis, treatment, or operation after introducing the medical instrument. Therefore, it is difficult for the conventional arts to accurately show an anatomical position of a medical instrument in a living body.

The present invention has been achieved in view of the above circumstances, and an object of the present invention is to provide a medical guiding system capable of making a position of the medical instrument which is introduced into a living body targeting a region of interest formed of at least either an organ or a tissue in the living body with respect to the region of interest on a guide image coincide with an actual position of the medical instrument with respect to the region of interest, with high accuracy.

SUMMARY OF THE INVENTION

A medical guiding system according to the present invention comprises: a detection portion for detecting at least one of a position and an orientation of a medical instrument; a storage portion for storing a plurality of reference image data, the plurality of reference image data being obtained from a human body in a plurality of states before the medical instrument is used with respect to a subject, and including anatomical positional information of at least one of a viscus and an organ that corresponds to each of the plurality of states; a state selection portion for selecting, among the plurality of reference image data stored in the storage portion, the reference image data obtained in a state coincident with or approximate to a state of the subject in using the medical instrument; and a guide image creation portion for creating a guide image showing at least one of an anatomical position, shape, and orientation of the medical instrument with respect to the subject, based on at least one of the position and the orientation of the medical instrument detected by the detection portion and the reference image data stored in the state selection portion.

The above and other objects, features and advantages of the invention will become more clearly understood from the following description referring to the accompanying drawings.

BRIEF DESCRIPTION OF THE DRAWINGS

FIG. 1 relates to a first embodiment of the present invention and is a block diagram showing a configuration of an ultrasound diagnostic apparatus.

FIG. 2 is an illustration diagram typically showing a body surface detection coil in an example of use.

FIG. 3 is a lateral view showing a body cavity contact probe.

FIG. 4 is a block diagram showing a configuration of an image processing apparatus.

FIG. 5 is an illustration diagram showing reference image data stored in a reference image storage portion.

FIG. 6 is an illustration diagram showing a voxel space.

FIG. 7 is an illustration diagram showing a key arrangement of a keyboard.

FIG 8 is an illustration diagram showing an orthogonal base with an origin set on a transmission antenna to express position/orientation data.

FIG. 9 is an illustration diagram showing a situation in which body cavity feature point on a subject side are mapped in a voxel space.

FIG. 10 is an illustration diagram showing a situation in which image index data is created by an image index creation circuit.

FIG. 11 is an illustration diagram showing a situation in which insertion shape data is created by the insertion shape creation circuit.

FIG. 12 is an illustration diagram showing three-dimensional human body image data.

FIG. 13 is an illustration diagram showing a situation in which the image index data and the insertion shape data are filled in a voxel space in a synthesis memory by a synthesis circuit.

FIG. 14 is an illustration diagram showing three-dimensional guide image data in a case where the subject is observed from a ventral side.

FIG. 15 is an illustration diagram showing a three-dimensional guide image data in a case where the subject is observed from the same direction as that of an ultrasound tomographic image.

FIG. 16 is a diagram showing a three-dimensional guide image and an ultrasound tomographic image displayed on a display apparatus.

FIG. 17 is a flowchart showing processing contents as a whole.

FIG. 18 is a flowchart showing specific contents a body surface feature points and body cavity feature point specification processing on the reference image of FIG. 17.

FIG. 19 is a flowchart showing specific processing contents of a correction value calculation processing in FIG 17.

FIG. 20 is an illustration diagram of the processing in FIG. 19.

FIG. 21 is a flowchart showing a specific processing content of an ultrasound tomographic image and three-dimensional guide image creation/display processing in FIG. 17.

FIG. 22 is an illustration diagram showing one example of supine position reference image data.

FIG. 23 is an illustration diagram showing one example of left lateral position reference image data.

FIG. 24 relates to a second embodiment of the present invention and is an illustration diagram showing a key arrangement of a keyboard.

FIG. 25 relates to a third embodiment of the present invention and is an illustration diagram showing a key arrangement of a keyboard.

FIG. 26 is an illustration diagram showing rotation and movement of three-dimensional human body image data.

FIG. 27 is an illustration diagram showing correspondence of combination between the keys and reference image data.

FIG. 28 relates to a fourth embodiment of the present invention and is an illustration diagram showing synthesis/deformation of the three-dimensional human body image data.

FIG. 29 is an illustration diagram showing correspondence between combination of keys and the three-dimensional human body image data.

FIG. 30 is a block diagram showing a configuration of an image processing apparatus.

DETAILED DESCRIPTION OF PREFERRED EMBODIMENTS

Hereinafter, description will be made on the present invention with reference to the illustrated embodiments. In the embodiments below, description will be made assuming that an ultrasound endoscope is used as a medical instrument and a medical guiding system creates a guide image based on which operation of the ultrasound endoscope is assisted. Note that shapes, ratio of sizes, or arrangement position of the components, and the like of the present invention are not limited to those illustrated in the drawings.

(First Embodiment)

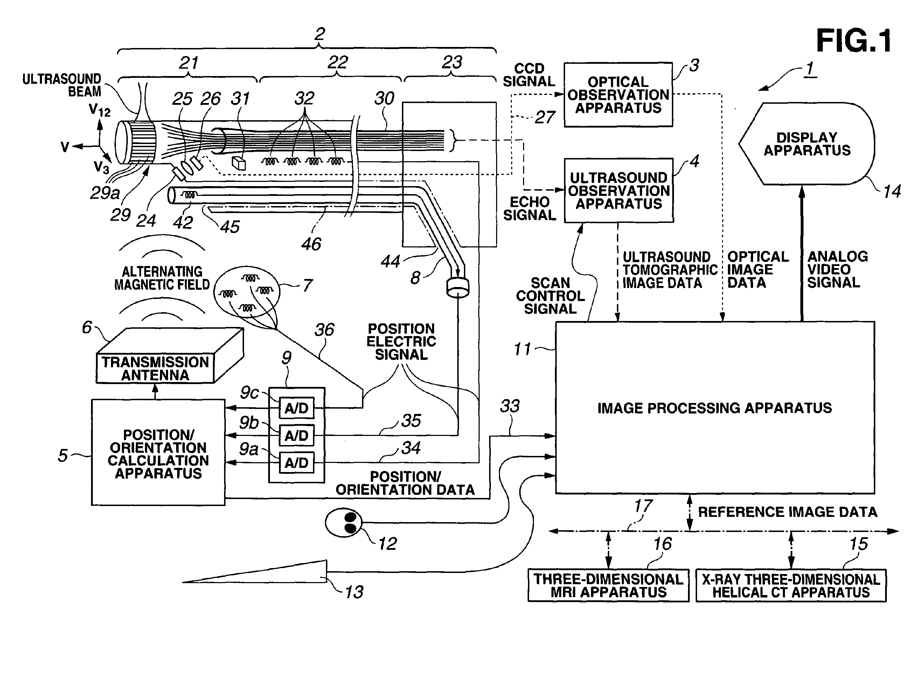

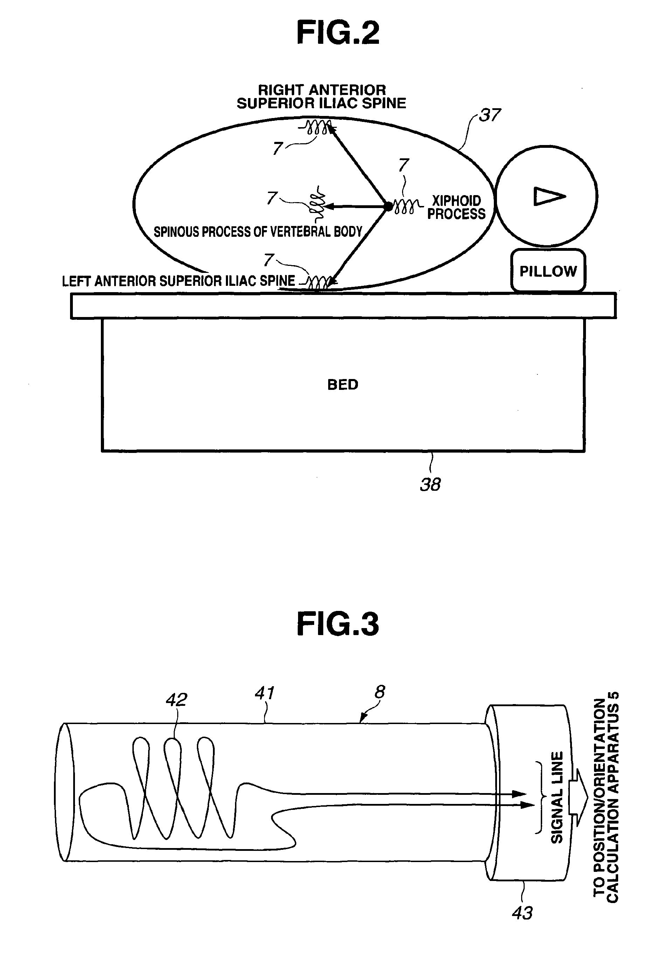

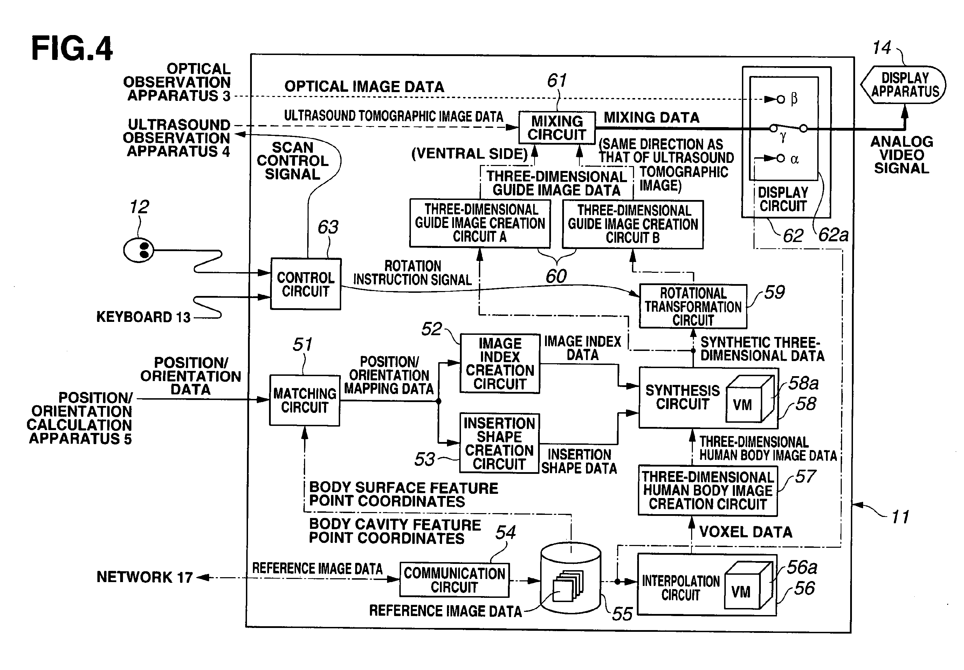

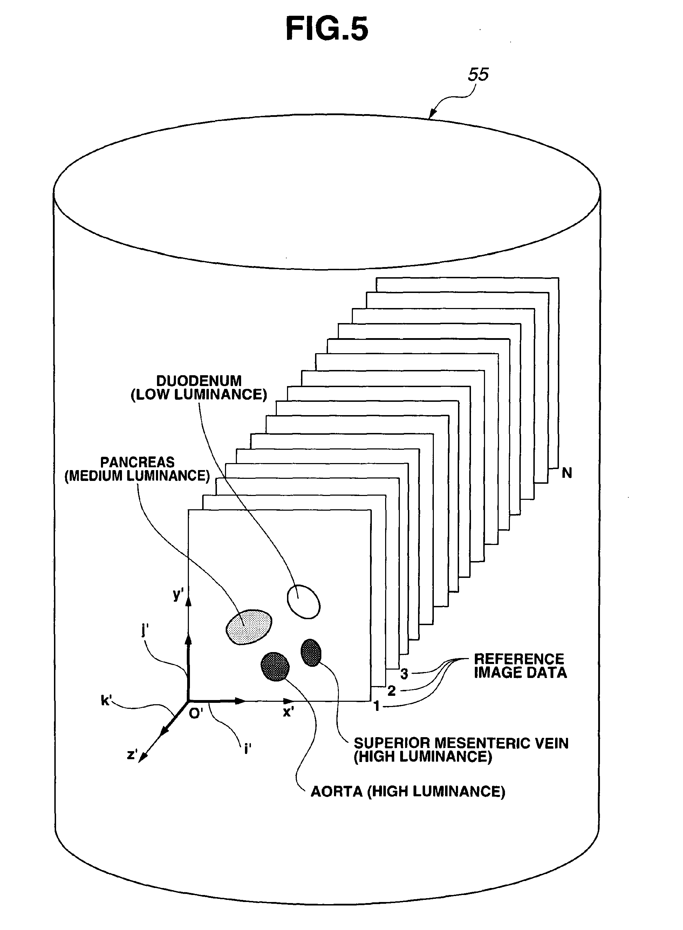

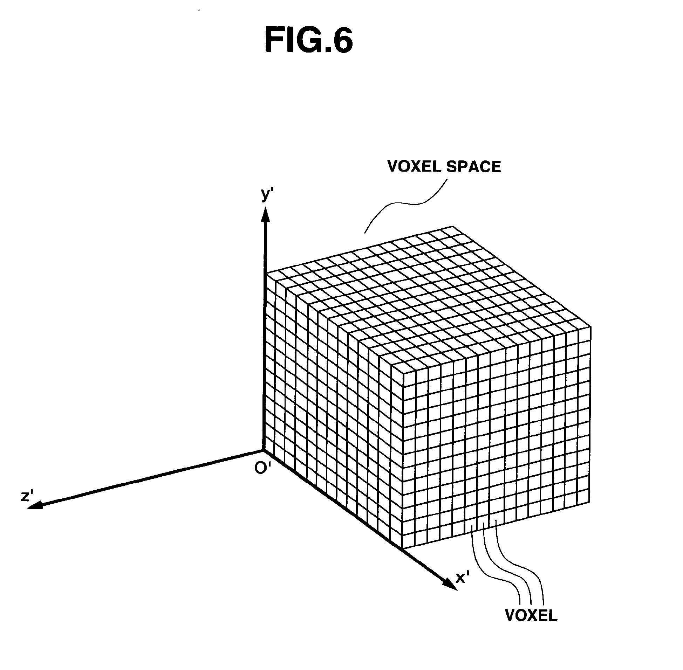

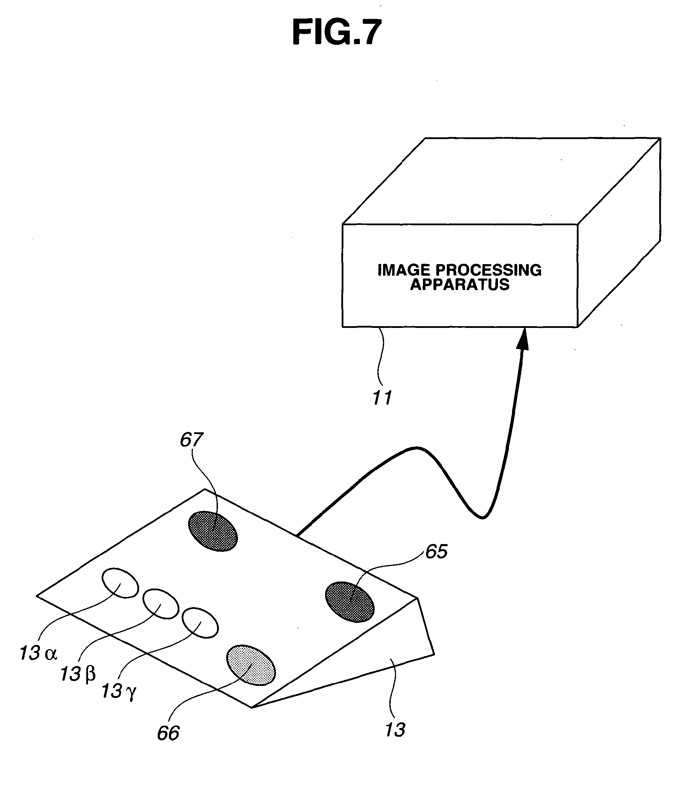

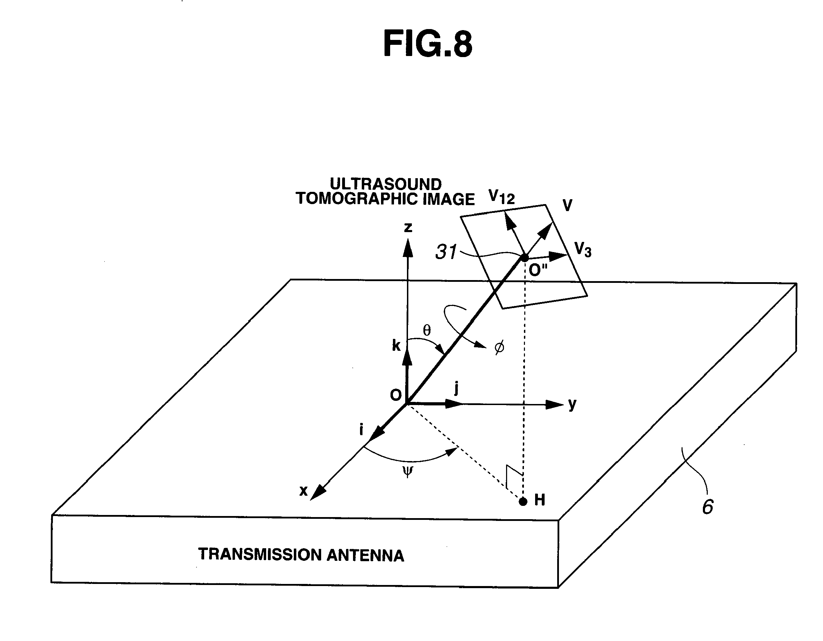

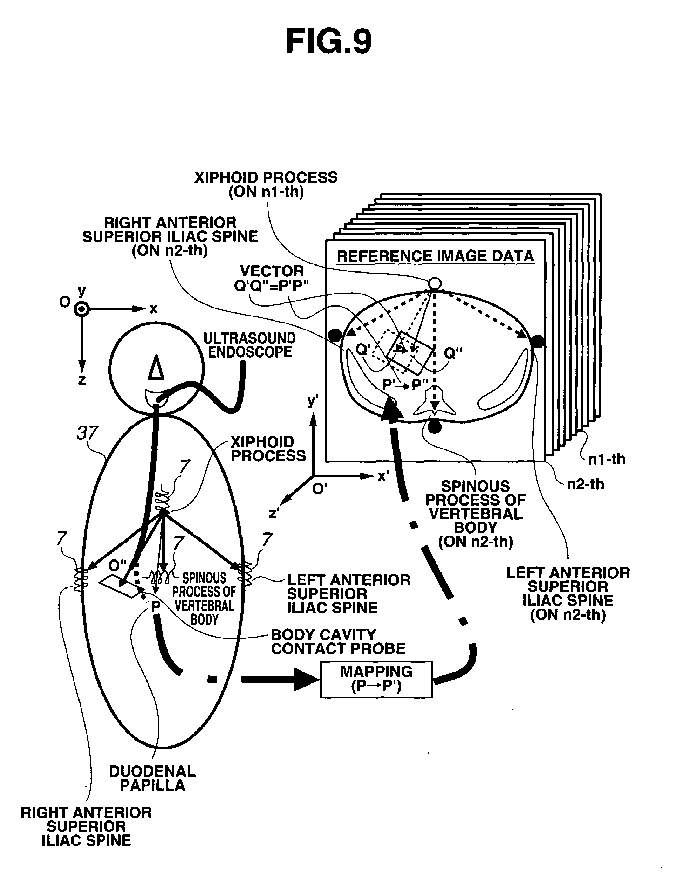

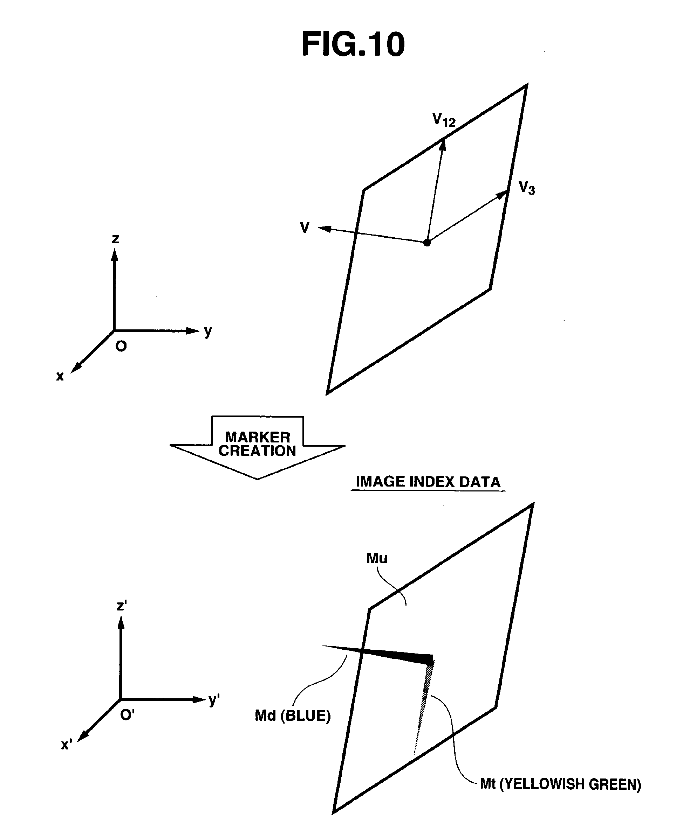

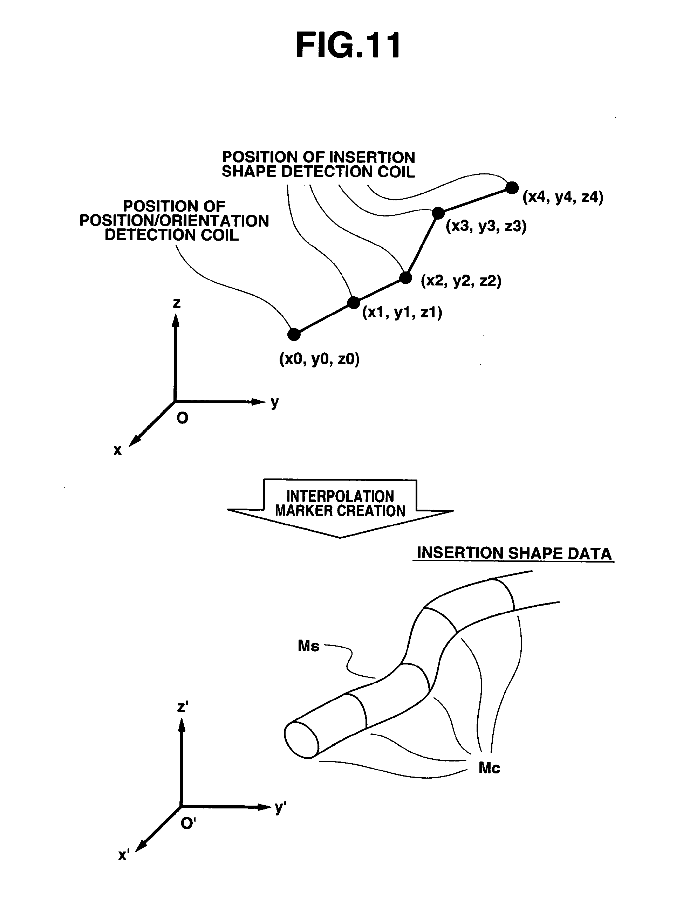

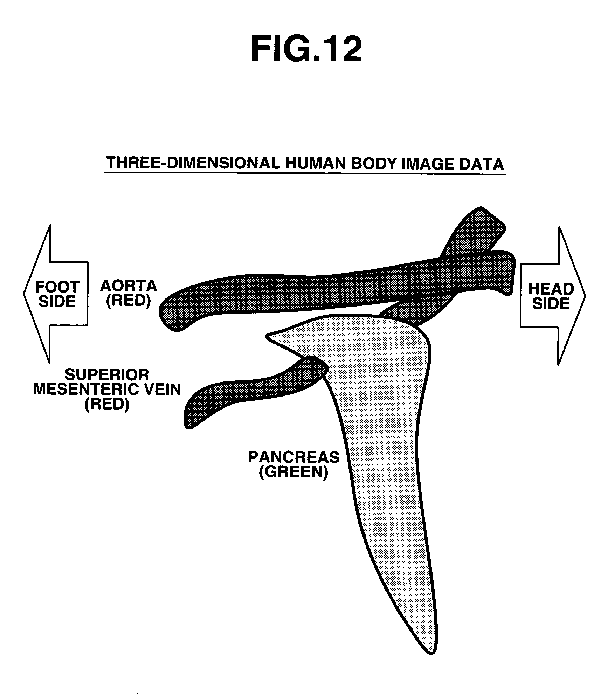

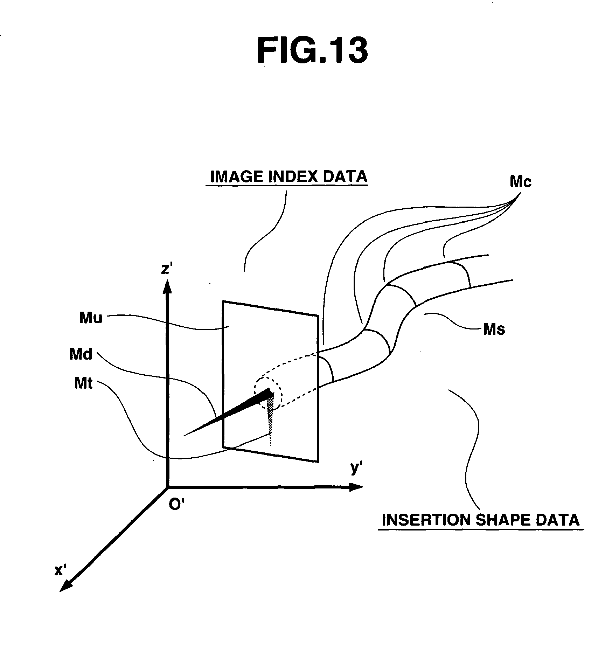

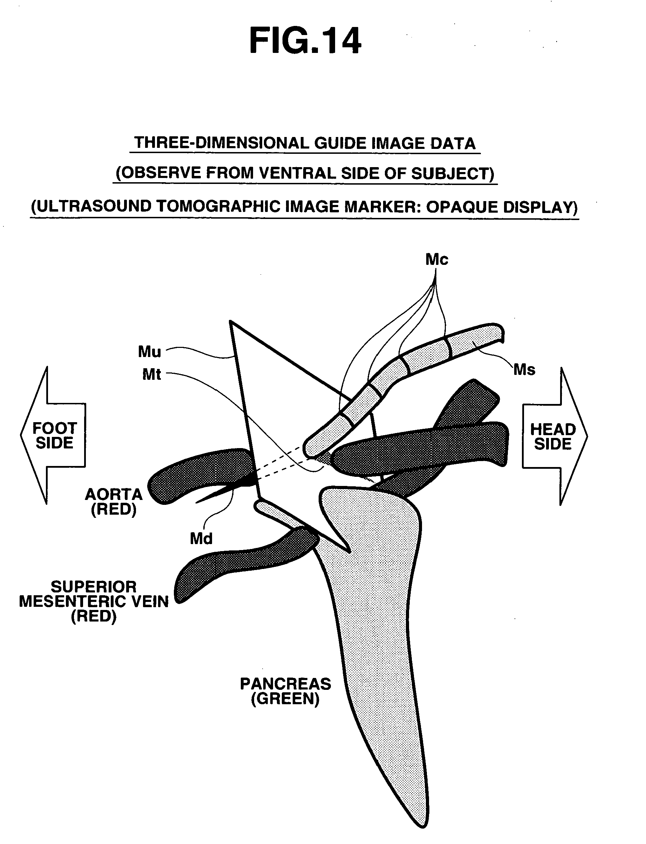

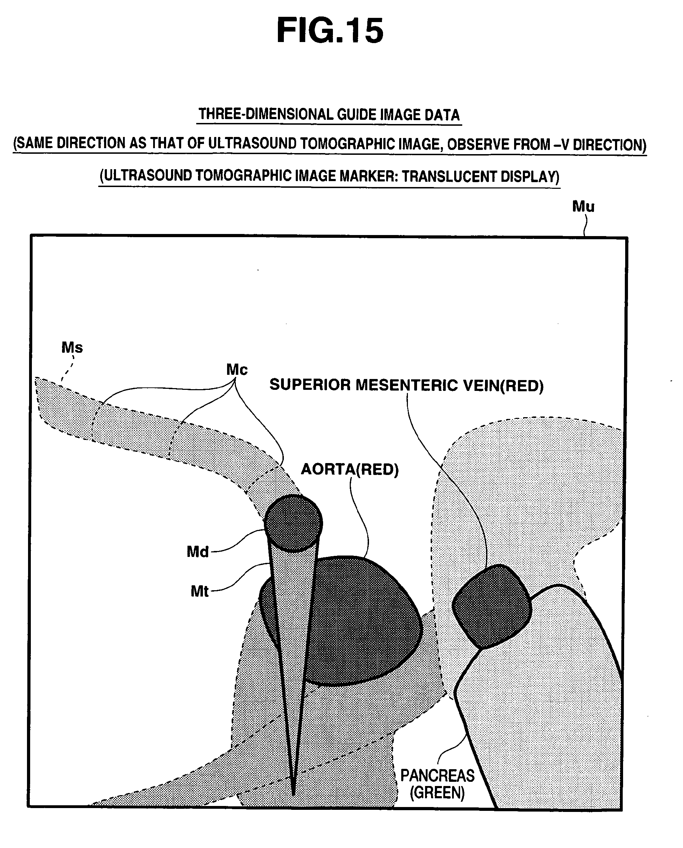

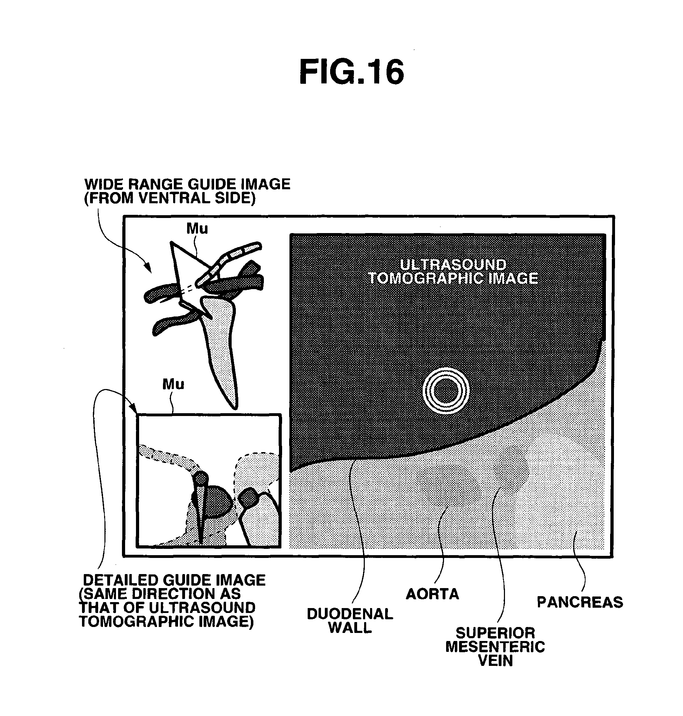

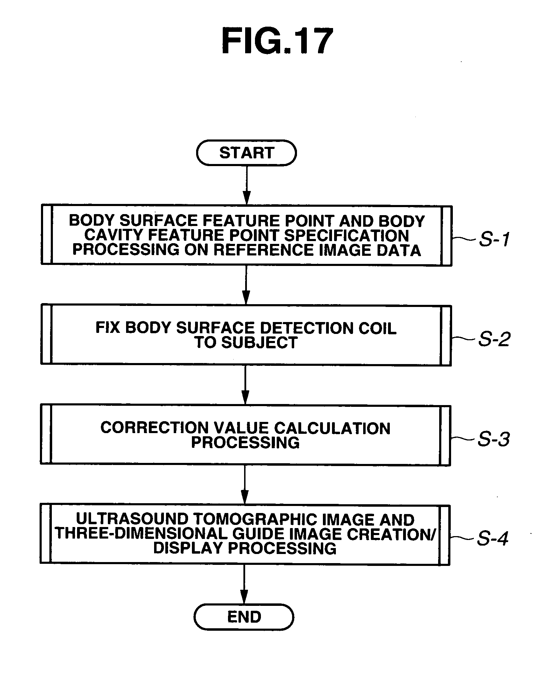

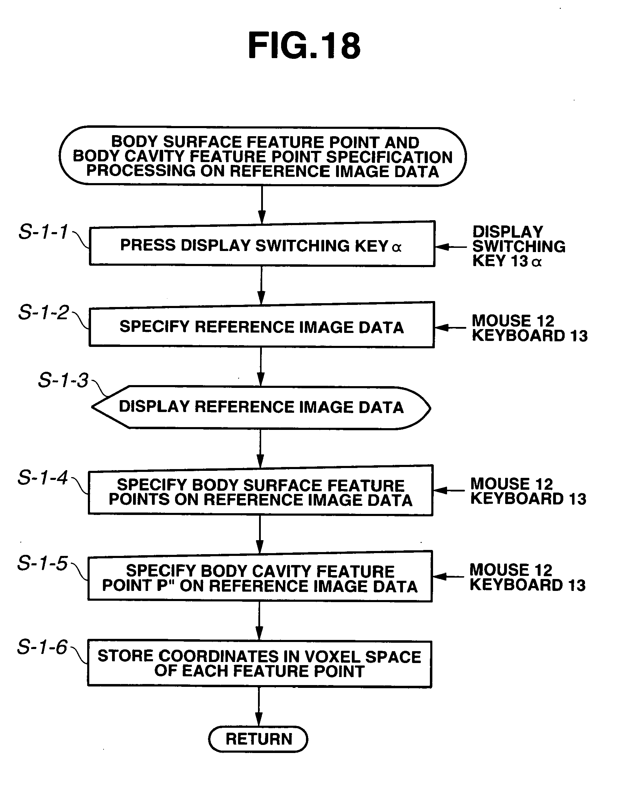

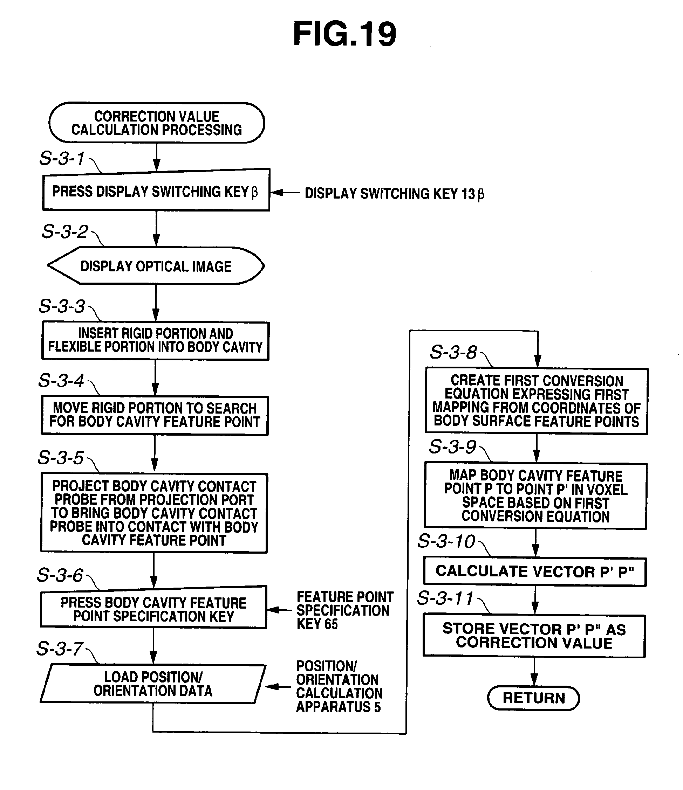

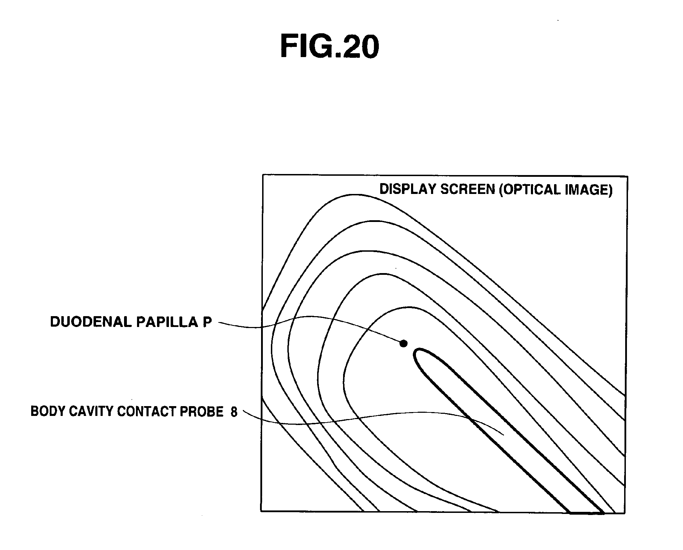

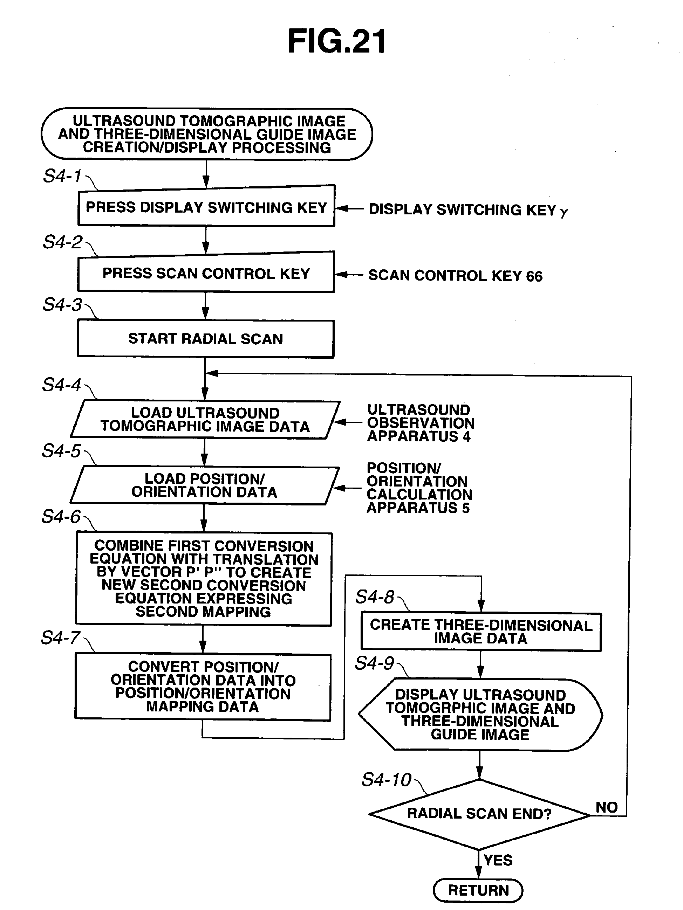

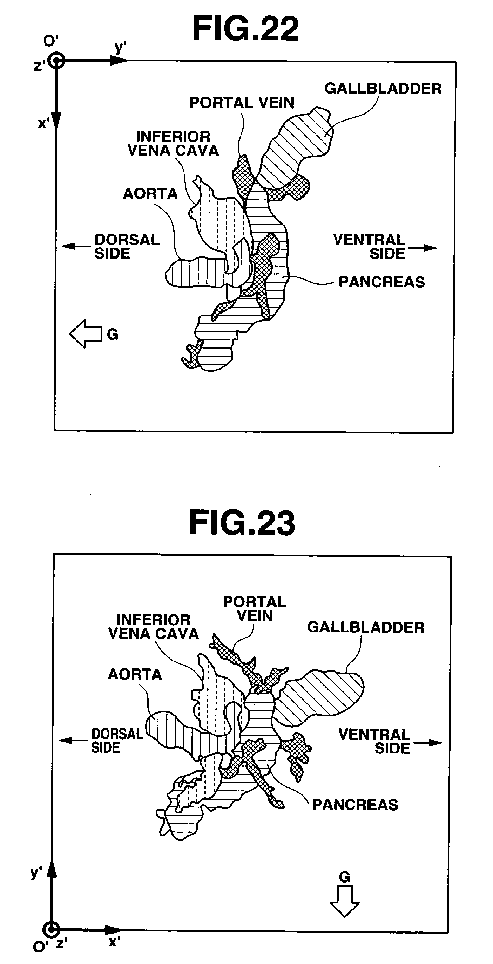

FIGS. 1 to 23 relate to the first embodiment of the present invention, in which: FIG. 1 is a block diagram showing a configuration of an ultrasound diagnostic apparatus; FIG. 2 is an illustration diagram typically showing a body surface detection coil in an example of use; FIG. 3 is a lateral view showing a body cavity contact probe; FIG 4 is a block diagram showing a configuration of an image processing apparatus; FIG. 5 is an illustration diagram showing reference image data stored in a reference image storage portion; FIG. 6 is an illustration diagram showing a voxel space; FIG. 7 is an illustration diagram showing a key arrangement of a keyboard; FIG. 8 is an illustration diagram showing an orthogonal base with an origin set on a transmission antenna to express position/orientation data; FIG. 9 is an illustration diagram showing, for example, a situation in which body cavity feature points on a subject side are mapped in a voxel space; FIG. 10 is an illustration diagram showing a situation in which image index data is created by an image index creation circuit; FIG. 11 is an illustration diagram showing a situation in which insertion shape data is created by the insertion shape creation circuit; FIG. 12 is an illustration diagram showing three-dimensional human body image data; FIG. 13 is an illustration diagram showing a situation in which the image index data and the insertion shape data are filled in a voxel space in a synthesis memory by a synthesis circuit; FIG. 14 is an illustration diagram showing three-dimensional guide image data in a case where the subject is observed from a ventral side; FIG. 15 is an illustration diagram showing a three-dimensional guide image data in a case where the subject is observed from the same direction as that of an ultrasound tomographic image; FIG. 16 is a diagram showing a three-dimensional guide image and an ultrasound tomographic image displayed on a display apparatus; FIG. 17 is a flowchart showing processing contents as a whole; FIG. 18 is a flowchart showing specific processing contents of a body surface feature points and body cavity feature points specification processing on the reference image of FIG. 17; FIG. 19 is a flowchart showing specific processing contents of a correction value calculation processing in FIG. 17; FIG. 20 is an illustration diagram of the processing in FIG. 19; FIG. 21 is a flowchart showing specific processing contents of an ultrasound tomographic image and three-dimensional guide image creation/display processing in FIG. 17; FIG. 22 is an illustration diagram showing one example of supine position reference image data; and FIG. 23 is an illustration diagram showing one example of left lateral position reference image data.

A medical guiding system 1 of the present embodiment is incorporated in an ultrasound diagnostic apparatus and serves as a system for performing guiding to assist introduction of an ultrasound endoscope 2 as a medical instrument into a body of a subject. The medical guiding system 1 of the present embodiment includes the ultrasound endoscope 2, an ultrasound observation apparatus 4, a position/orientation calculation apparatus 5, an image processing apparatus 11, a display apparatus 14, an optical observation apparatus 3, and an input apparatus, and the apparatuses are connected by wired or wireless communication means.

In addition, the medical guiding system 1 is connected to a network 17 using an optical fiber, an electrical cable, or wireless communication and the like, which is provided outside of the medical guiding system 1. The network 17 is connected with an X-ray three-dimensional helical CT apparatus (X-ray 3-dimensional computed tomography system) 15 and a three-dimensional MRI apparatus (3-dimensional magnetic resonance imaging system) 16. The medical guiding system 1 is capable of transmitting and receiving data to and from the X-ray three-dimensional helical CT apparatus 15 and the three-dimensional MRI apparatus 16.

The ultrasound endoscope 2 includes: a rigid portion 21 configured of a rigid material such as stainless steel at a distal end; a long flexible portion 22 configured of a flexible material on rear end side of the rigid portion 21; and an operation portion 23 configured of a rigid material on rear end side of the flexible portion 22 so as to be inserted and used in a body of a subject, such as esophagus, stomach, duodenum and the like. Note that the rigid portion 21 and the flexible portion 22 form an insertion portion to be introduced into the body of the subject.

The rigid portion 21 is provided with an optical observation window 24 formed of a cover glass, an objective lens 25 which forms an optical image on an inner side of the optical observation window 24, and for example a CCD (Charge Coupled Device) 26 as an image pickup device disposed at an image-forming position of the objective lens 25. Furthermore, adjacent to the optical observation window 24 is provided an illumination light irradiation window (illumination window), not shown, from which illumination light is irradiated into a body cavity.

The CCD 26 is connected to the optical observation apparatus 3 by a signal line 27. The illumination light irradiation window not shown is configured such that illumination light is irradiated therefrom to illuminate inside of the body cavity. An image of the body cavity surface is formed on the CCD 26 via from the optical observation window 24 to the objective lens 25, and the CCD signal from the CCD 26 is outputted to the optical observation apparatus 3 via the signal line 27.

The rigid portion 21 has, for example, a columnar distal end part with a group of ultrasound transducers cut into small pieces like strips and arranged in an annular and array shape around the insertion axis of the rigid portion, and the group of ultrasound transducers forms an ultrasound transducer array 29. Each of the ultrasound transducers 29a configuring the ultrasound transducer array 29 is connected to the ultrasound observation apparatus 4 via the operation portion 23 through a signal line 30. An annulus center of the ultrasound transducer array 29 is a pivot center of an ultrasound beam by a radial scan to be described later.

Note that, hereinafter it is assumed that a surface to be scanned by the ultrasound transducer array 29 is called as a scanning surface. Fixed orthonormal bases (unit vectors of the respective directions) V, V3, and V12 fixed to the rigid portion 21 are defined as shown in FIG. 1. That is, the base vector V is defined to be parallel to an insertion axis direction of the rigid portion 21, in other words, a normal direction vector of the scanning surface, and in a case where a predetermined scan direction of the ultrasound transducer array 29 is assumed to be the twelve o'clock direction, the base vector to orient in the three o'clock direction is defined as V3, and the base vector to orient in the twelve o'clock direction is defined as V12. Note that, though vectors are normally described in bold italics, the vectors are denoted in normal alphanumeric characters in the present embodiment.

In the rigid portion 21, an image position/orientation detection coil 31 serving as an image position/orientation detection element with respect to the ultrasound transducer array 29 is fixedly provided in the immediate vicinity of the annulus center of the ultrasound transducer array 29. The image position/orientation detection coil 31 includes integrally formed coils which are wound in two axes directions so as to orient in two directions (axes) of the vectors V and V3, and is set so as to be able to detect both directions of vectors V and V3.

In the flexible portion 22 are provided a plurality of insertion shape detection coils 32, for example, at predetermined intervals along an insertion axis in order to detect an insertion shape of the flexible portion 22 configuring the insertion portion of the ultrasound endoscope 2. As shown in FIG. 1, the insertion shape detection coils 32 are coils wound in one axis direction and fixed in the inside of the flexible portion 22 such that a winding axis direction of the coils coincides with the insertion axis direction of the flexible portion 22.

Note that the position and orientation of the rigid portion 21 can be detected from the position of the image position/orientation detection coil 31. In addition, a bendable bending portion is often provided in the vicinity of the distal end of the flexible portion 22, and the plurality of insertion shape detection coils 32 may be provided only in the vicinity of the bending portion to detect the insertion shape of the distal end side part of the insertion portion of the ultrasound endoscope 2.

In the present embodiment, by employing the plurality of insertion shape detection coils 32, the insertion shape is detected by using magnetic fields. This configuration prevents an operator and a patient (subject) from being exposed to radiation in detecting insertion shape.

The position/orientation calculation apparatus 5 is for detecting the position and the orientation of the image position/orientation detection coil 31 and the positions of the plurality of insertion shape detection coils 32, and the like, and is connected to the transmission antenna 6, an A/D unit 9 including a plurality of A/D units 9a, 9b, and 9c, and the image processing apparatus 11 by signal lines. The position/orientation calculation apparatus 5 and the image processing apparatus 11 are electrically connected with each other through a cable 33 of the RS-232C standard, for example.

The transmission antenna 6 is configured of a plurality of transmission coils of different winding axes orientation, not shown, and these transmission coils are integrally stored in a rectangular parallelepiped case, for example. Each of the plurality of transmission coils is connected to the position/orientation calculation apparatus 5.

Each of the A/D units 9a, 9b, and 9c includes an amplifier, not shown, for amplifying inputted analog signals and an analog/digital conversion circuit, not shown, for converting the amplified signals into digital data by sampling the signals. The A/D unit 9a is connected individually to the image position/orientation detection coil 31 and each of the plurality of insertion shape detection coils 32 by a signal line 34. The A/D unit 9b is connected to a long body cavity contact probe 8 by a signal line 35. The A/D unit 9c is connected individually to each of a plurality of body surface detection coils 7 by a signal line 36.

As shown in FIG. 2, the body surface detection coils 7 include four coils each of which is wound in one axis direction, and the coils are detachably fixed on a body surface of a subject 37, more specifically on feature points on the abdominal body surface (hereinafter only called as body surface feature points), by a tape, belt, band, adhesive or negative pressure absorption, and the like, and used for position detection of the body surface feature points using the magnetic fields. FIG. 2 illustrates the subject in the left lateral position, because, in normal upper endoscopy, the subject 37 lies on a bed 38 with the left side downward, in the so-called left lateral position, and the insertion portion of the ultrasound endoscope 2 is inserted from the mouth.

The present embodiment is described with the body surface feature points taking four characteristics points on a skeleton, that is, "xiphoid process", "left anterior superior iliac spine" on the left side of the pelvis, "right anterior superior iliac spine" on the right side of the pelvis, and "spinous process of vertebral body" in the middle of the left and right anterior superior iliac spines on the spine. The positions of these four points can be specified by operator's palpation. In addition, these four points are not located on the same plane, and form oblique coordinate system with the xiphoid process set as the origin and with the three vectors directed to other feature points as fundamental vectors. The oblique coordinate system is shown by the heavy line in FIG. 2.

FIG. 3 shows the body cavity contact probe 8. The body cavity contact probe 8 includes an outer cylinder 41 configured of a flexible material. A body cavity detection coil 42 is fixedly provided in a distal end of the outer cylinder 41 and a connector 43 is provided at a rear end of the outer cylinder 41.

As shown in FIG. 3, the body cavity detection coil 42 is a coil wound in one axis direction and fixed to the distal end of the body cavity contact probe 8. The body cavity detection coil 42 is fixed such that the winding axis direction thereof coincides with the insertion axis direction of the body cavity contact probe 8. In addition, the body cavity detection coil 42 is used for detecting a position of a region of interest or the like in the body cavity with which the distal end of the body cavity contact probe 8 comes into contact.

As shown in FIG. 1, the ultrasound endoscope 2 includes a tubular treatment instrument channel 46 from the operation portion 23 through the flexible portion 22 to the rigid portion 21. The treatment instrument channel 46 is provided at the operation portion 23 with a treatment instrument insertion port (hereinafter abbreviated as a forceps port for simplification) 44, as a first opening, to which a forceps and the like is inserted, and at the rigid portion 21 with a projection port 45 as a second opening.

The treatment instrument channel 46 is configured such that the body cavity contact probe 8 can be inserted from the forceps port 44 and projected from the projection port 45. An opening direction of the projection port 45 is set such that the body cavity contact probe 8 enters within an optical field of view range of the optical observation window 24 when the body cavity contact probe 8 is projected from the projection port 45.

As shown in FIG. 4, the image processing apparatus 11 includes: a matching circuit 51; an image index creation circuit 52; an insertion shape creation circuit 53; a communication circuit 54; a reference image storage portion 55 as a reference data retaining portion to retain reference data; an interpolation circuit 56; a three-dimensional human body image creation circuit 57 as a human body image creation portion; a synthesis circuit 58 as a synthesis portion; a rotational transformation circuit 59; three-dimensional guide image creation circuits 60 (hereinafter described as three-dimensional guide image creation circuit A and three-dimensional guide image creation circuit B) as guide image creation portions for creating three-dimensional guide images in two different eye directions; a mixing circuit 61; a display circuit 62; and a control circuit 63. The communication circuit 54 includes a high-capacity and high-speed communication apparatus, and is connected to the X-ray three-dimensional helical CT apparatus 15 and the three-dimensional MRI apparatus 16 via the network 17.

The matching circuit 51 is inputted with position/orientation data outputted from the position/orientation calculation apparatus 5, and as described later, maps the position/orientation data calculated on the orthogonal coordinate axis O-xyz according to a predetermined conversion equation to calculate new position/orientation data on the orthogonal coordinate axis O'-x'y'z'. Then, the matching circuit 51 outputs the new position/orientation data to the image index creation circuit 52 for creating image index data and the insertion shape creation circuit 53 for creating insertion shape data, as position/orientation mapping data.

The reference image storage portion 55 is composed of a hard disk drive and the like capable of saving a large volume of data. The reference image storage portion 55 stores a plurality of reference image data as anatomical image information.

As shown in FIG. 5, the reference image data is tomographic image data of the subject 37 obtained from the X-ray three-dimensional helical CT apparatus 15, the three-dimensional MRI apparatus 16, or other ultrasound diagnostic appatatuses, through the network 17. In the present embodiment, for convenience of description, the reference image data is assumed to be obtained from one specific subject among a plurality of subjects and to be data of tomographic images of squares with several tens of centimeters on a side which are perpendicular to the body axis (axis extending from the subject's head to feet) and which have a pitch of 0.5 mm to several mm.

The reference image data in the reference image storage portion 55 in FIG. 5 are denoted by reference numerals 1 to N for the convenience of description. Here, as shown in FIG. 5, an orthogonal coordinate axis O'-x'y'z' and orthonormal bases therefor (unit vectors in the respective axis directions) i', j', and k', which are fixed with respect to a plurality of reference image data, are defined on the reference image data with an origin O' defined at a lower leftmost position of the 1 st reference image data.

As shown in FIG. 4, each of the interpolation circuit 56 and the synthesis circuit 58 incorporates a volume memory VM. For the convenience of the description, hereinafter the volume memories provided to the interpolation circuit 56 and the synthesis circuit 58 are referred to as an interpolation memory 56a and a synthesis memory 58a, respectively.

Each of the volume memories VM is configured to be able to store a large volume of data. A voxel space is assigned to a partial storage region of each of the volume memories VM. As shown in FIG. 6, the voxel space is composed of memory cells (hereinafter referred to as voxels) having addresses corresponding to the orthogonal coordinate axis O'-x'y'z'.

The three-dimensional human body image creation circuit 57 for creating the three-dimensional human body image and the rotational transformation circuit 59 for performing rotational transformation which are shown in FIG. 4 incorporate a high-speed processor, not shown, that performs high-speed image processing such as extraction of voxels and pixels by luminance, rotational transformation, similarity transformation, and parallel translation.

The display circuit 62 has a switch 62a for switching input thereof. The switch 62a has input terminals α, β, and γ, and one output terminal. The input terminal α is connected to the reference image storage portion 55. The input terminal β is connected to an output terminal not shown of the optical observation apparatus 3. The input terminal γ is connected to the mixing circuit 61. The output terminal is connected to the display apparatus 14, which displays optical images, ultrasound tomographic images, and three-dimensional guide images, and the like.

The control circuit 63 is connected to the respective portions and circuits in the image processing apparatus 11 via signal lines not shown so as to output instructions to the portions and circuits. The control circuit 63 is connected directly to the ultrasound observation apparatus 4, a mouse 12, and a keyboard 13 via control lines.

As shown in FIG. 7, the keyboard 13 has a body cavity feature point specification key 65, a scan control key 66, display switching keys 13α, 13β, and 13γ, and a body position selection key 67.

Depressing any of the display switching keys 13α, 13β, and 13γ causes the control circuit 63 to output an instruction to the display circuit 62 to switch the switch 62a to the input terminal α, β, or γ. Depressing the display switching key 13α allows the switch 62a to be switched to the input terminal α. Depressing the display switching key 13β allows the switch 62a to be switched to the input terminal β. Depressing the display switching key 13γ allows the switch 62a to be switched to the input terminal γ.

Next, functions of the medical guiding system 1 of the present embodiment including the above-described configurations will be described with reference to FIGS. 1 and 4. Each of the arrow lines in FIGS. 1 and 4 shows flows of signals and data described below.

(a) First: flows of signals and data related to optical images, which are indicated by dotted lines.
(b) Second: flows of signals and data related to ultrasound tomographic images, which are indicated by dashed lines.
(c) Third: flows of signals and data related to positions and flows of data created by processing the signals and data, which are indicated by solid lines.
(d) Fourth: flows of reference image data and data created by processing the reference image data, which are indicated by dashed-dotted lines.
(e) Fifth: flows of signals and data related to a final display screen obtained by synthesizing ultrasound tomographic image data (described below) with three-dimensional guide image data (described below), which are indicated by thick lines.
(f) Sixth: flows of signals and data related to other controls, which are indicated by curved lines.

Hereinafter, description will be sequentially made along the flows of the signals and data shown in FIGS. 1 and 4.

(a) The flows of signals and data related to optical images

From the illumination light irradiation window, not shown, of the rigid portion 21 is irradiated illumination light to optical filed of view range side. The CCD 26 picks up an image of an object within the optical field of view range to photoelectrically convert the picked-up image and generates a CCD signal. The CCD 26 then outputs the CCD signal to the optical observation apparatus 3. The optical observation apparatus 3 creates the data of the image within the optical filed of view range based on the inputted CCD signal. The optical observation apparatus 3 then outputs the data as optical image data to the input terminal β of the switch 62a of the display circuit 62 in the image processing apparatus 11.

(b) The flows of signals and data related to ultrasound tomographic images

When the operator depresses the scan control key 66 as a condition input portion through which conditions for selecting scan information are inputted, the control circuit 63 outputs a scan control signal to instruct on/off control of radial scan to be described later to the ultrasound observation apparatus 4. The ultrasound observation apparatus 4 which has received the scan control signal selects some ultrasound transducers 29a among the ultrasound transducers 29a configuring the ultrasound transducer array 29, to transmit excitation signals having a shape like pulse voltages to the selected ultrasound transducers. The selected some ultrasound transducers 29a receive the excitation signals to convert the signals into ultrasounds that are longitudinal waves in a medium.

At this time, the ultrasound observation apparatus 4 delays the excitation signals so that the excitation signals reach the respective ultrasound transducers 29a at different times. The value (delay amount) of the delay is adjusted so that ultrasounds excited by the ultrasound transducers 29a form one ultrasound beam when overlapped one another in the subject 37.

The ultrasound beam is irradiated to the exterior of the ultrasound endoscope 2, and a reflected wave from the interior of the subject 37 returns to each ultrasound transducer 29a via a path opposite to that of the ultrasound beam. Each ultrasound transducer 29a converts the reflected wave into an electrical echo signal and transmits the echo signal to the ultrasound observation apparatus 4 via a path opposite to that of the excitation signal.

The ultrasound observation apparatus 4 reselects a plurality of ultrasound transducers 29a to be involved in the formation of an ultrasound beam such that the ultrasound beam pivots in a plane (hereinafter referred to as a radial scanning surface) which contains the center of the annulus of the ultrasound transducer array 29 and which is perpendicular to the rigid portion 21 and flexible portion 22, and then transmits excitation signals again to the selected ultrasound transducers 29a. The transmission angle of the ultrasound beam thus changes. By repeatedly performing such processings, so-called radial scan can be achieved.

At this time, the ultrasound observation apparatus 4 creates a piece of digitalized ultrasound tomographic image data perpendicular to the insertion axis of the rigid portion 21 for one radial scan with respect to the ultrasound transducer array 29, based on the echo signal converted from the reflected wave by the ultrasound transducers 29a, and then outputs the created ultrasound tomographic image data to the mixing circuit 61 in the image processing apparatus 11. At this time, the ultrasound observation apparatus 4 creates the ultrasound tomographic image data by processing the data into a square.

Thus in the present embodiment, the ultrasound observation apparatus 4 reselects a plurality of ultrasound transducers 29a to be involved in the formation of an ultrasound beam to transmit excitation signals again, so that the twelve o'clock direction of the square ultrasound tomographic image, for example, is determined depending on which of the ultrasound transducers 29a the ultrasound observation apparatus 4 selects as the twelve o'clock direction and transmits excitation signals. Thus, the normal direction vector V, three o'clock direction vector V3, and twelve o'clock direction vector V12 of the ultrasound tomographic image are defined. In addition, the ultrasound observation apparatus 4 creates ultrasound tomographic image data through observations from -V direction opposite to that of the normal vector V.

The radial scan by the ultrasound transducer array 29 and the creation and output to the mixing circuit 61 of ultrasound tomographic image data by the ultrasound observation apparatus 4 are performed in real time.

(c) The flows of signals and data related to positions and the flows of data created by processing the signals and data

The position/orientation calculation apparatus 5 excites the transmission coil, not shown, in the transmission antenna 6. The transmission antenna 6 generates an alternating magnetic field in a space.

Two coils configuring the image position/orientation detection coil 31 wound in the directions of the vectors V and V3 and having orthogonal winding axes, the plurality of insertion shape detection coils 32, the body cavity detection coil 42, and the body surface detection coils 7 detect the alternating magnetic field to convert the detected magnetic fields into position electric signals and then output the signals to the A/D units 9a, 9b, and 9c.

Each of the A/D units 9a, 9b, and 9c amplifies the position electric signal using an amplifier to sample and convert the amplified signal into digital data using an analog/digital conversion circuit, and outputs the digital data to the position/orientation calculation apparatus 5.

Next, the position/orientation calculation apparatus 5 calculates, based on the digital data from the A/D unit 9a, the position of the image position/orientation detection coil 31 and directions of the orthogonal winding axes thereof, that is, vectors V, V3. Then, the position/orientation calculation apparatus 5 calculates the outer product V x V3 of the vectors V and V3 corresponding to the directions of the orthogonal winding axes, thereby calculating the twelve o'clock vector V12 corresponding to the remaining orthogonal direction. Thus, the position/orientation calculation apparatus 5 calculates the orthogonal three directions, that is, the vectors V, V3, and V12.

Next, the position/orientation calculation apparatus 5 calculates, based on the digital data from the A/D units 9a to 9c, the respective positions of the plurality of insertion shape detection coils 32, the body surface detection coils 7, and the body cavity detection coil 42. Then, the position/orientation calculation apparatus 5 outputs, to the matching circuit 51 in the image processing apparatus 11, the position and orientation of the image position/orientation detection coil 31, the respective positions of the plurality of the insertion shape detection coils 32, the respective positions of the four body surface detection coils 7, and the position of the body cavity detection coil 42, as position/orientation data.

The position/orientation data will be described in detail below.

In the present embodiment, as shown in FIG. 8, the origin O is defiend on the transmission antenna 6, and the operator defines the orthogonal coordinate axis O-xyz and the orthonormal bases (unit vectors in the respective axis directions) i, j, and k on an actual space in which the operator inspects the subject 37. The position of the image position/orientation detection coil 31 is defined as O". Since the image position/orientation detection coil 31 is fixed in the immediate vicinity of the annulus center of the ultrasound transducer array 29, the position 0" coincides with the center of the radial scan and also with the center of ultrasound tomographic images.

Here, the position/orientation data is defined as follows.

The directional components of a position vector OO" at the position O" of the image position/orientation detection coil 31 on the orthogonal coordinate axis O-xyz:

(x0, y0, z0)

The angular components of an Euler angle (described later) indicating the orientation of the image position/ orientation detection coil 31 with respect to the orthogonal coordinate axis O-xyz:

$(\psi, \theta, \phi)$

The directional components of the position vector of each of the plurality of insertion shape detection coils 32 on the orthogonal coordinate axis O-xyz:

(xi, yi, zi) (i denotes a natural number from 1 to the total number of the insertion shape detection coils 32)

The directional components of the position vectors of the four body surface detection coils 7 on the orthogonal coordinate axis O-xyz:

(xa, ya, za), (xb, yb, zb), (xc, yc, zc), (xd, yd, zd)

The directional components of the position vector of the body cavity detection coil 42 on the orthogonal coordinate axis O-xyz:

(xp, yp, zp)

Here, the Euler angle is such an angle that, when the orthogonal coordinate axis O-xyz in FIG. 8 is added with rotations in this order around the z axis, the y axis, and the z axis again, directions of the respective axes align with each other as described below.

$$\text{i after the rotation} = V3, \text{ j after the rotation} = V12, \text{ and k after the rotation} = V$$

In addition, $\psi$ denotes the rotation angle around the z axis, $\theta$ denotes the rotation angle around the y axis, and $\phi$ denotes the rotation angle around the z axis again.

In FIG. 8, H denotes an intersecting point between an xy plane and a perpendicular line from the position O" to the xy plane. The angular components $(\psi, \theta, \phi)$ of the Euler angle correspond to the orientation of the image position/ orientation detection coil 31, that is, the orientation of the ultrasound tomographic image data.

The matching circuit 51 calculates, from the following first to fourth data groups, a conversion equation that maps a position and orientation expressed on the orthogonal coordinate axis O-xyz to a position and orientation in the voxel space expressed on the orthogonal coordinate axis O'-x'y'z'. The calculation method will be described later. In addition, the position/orientation data described in the first and the second data groups are changed by movement of the subject 37. The conversion equation is also newly created with the change of the body movement of the subject 37. The creation of a new conversion equation will also be described below.

The first data group among the position/orientation data includes the directional components (xa, ya, za), (xb, yb, zb), (xc, yc, zc), and (xd, yd, zd) of the position vectors on the orthogonal coordinate axis O-xyz, of the body surface detection coils 7 attached to the xiphoid process, the left anterior superior iliac spine, the right anterior superior iliac spine, and the spinous process of vertebral body of the subject 37, respectively. FIG. 9 shows the body surface detection coils 7 attached to the positions described above.

The second data group among the position/orientation data includes the directional components (xp, yp, zp) of the position vector of the body cavity detection coil 42 on the orthogonal coordinate axis O-xyz. In FIG. 9, the body cavity contact probe 8 incorporating at the distal end the body cavity detection coil 42 in a fixed manner is shown by the thick dotted lines.

The third data group includes the coordinates (xa', ya', za'), (xb', yb', zb'), (xc', yc', zc'), and (xd', yd', zd') on the orthogonal coordinate axis O'-x'y'z', of pixels on any of the 1st to N-th reference image data, which correspond to points closest to the body surface from each of the xiphoid process, the left anterior superior iliac spine, the right anterior superior iliac spine, and the spinous process of vertebral body. The pixels are previously specified by the operator on any of the 1st to N-th reference image data. The specifying method will be described later.

In FIG 9, these pixels are shown by black circles • and white circles o. The coordinates (xa', ya', za'), (xb', yb', zb'), (xc', yc', zc'), (xd', yd', zd') are read from the reference image storage portion 55 as the body surface feature point coordinates to the matching circuit 51, as shown in FIG. 4.

The fourth data group includes the coordinates (xp", yp", zp") on the orthogonal coordinate axis O'-x'y'z', of pixels on any of the 1st to N-th reference image data which corresponds to the duodenal papilla. These pixels are previously specified by the operator on any of the 1st to N-th reference image data. The specifying method will be described later.

The pixels are shown by P" in FIG. 9. The fourth coordinates (xp", yp", zp") of the pixels are read from the reference image storage portion 55 into the matching circuit 51 as the body cavity feature point coordinates as shown in FIG. 4.

Next, the matching circuit 51 maps the position/orientation data calculated on the orthogonal coordinate axis O-xyz according to the conversion equation and calculates new position/orientation data on the orthogonal coordinate axis O'-x'y'z'. Then, the matching circuit 51 outputs the new position/orientation data, as position/orientation mapping data, to the image index creation circuit 52 and the insertion shape creation circuit 53.

The image index creation circuit 52 creates image index data from the position/ orientation mapping data with a total of six degrees of freedom including the directional components $(x0, y0, z0)$ of the position vector OO" at the position O" of the image position and orientation detection coil 31, on the orthogonal coordinate axis O-xyz, and the angular components $(\psi, \theta, \phi)$ of the Euler angle indicating the orientation of the image position/orientation detection coil 31 with respect to the orthogonal coordinate axis O-xyz, and outputs the created image index data to the synthesis circuit 58.

This situation is shown in FIG. 10. That is, the image index data is created as shown in the lower part of the FIG. 10 from the position/orientation mapping data shown on the upper part of the FIG. 10. The image index data is image data on the orthogonal coordinate axis O'-x'y'z' in which a parallelogrammatic ultrasound tomographic image marker Mu is synthesized with, for example, a blue distal direction marker Md (expressed as blue in FIG. 10) and a yellowish green arrow-shaped 6 o'clock direction marker Mt (expressed as yellowish green in FIG. 10).

The insertion shape creation circuit 53 creates insertion shape data (through interpolation and marker creation processings) from the position/orientation mapping data of the directional components $(x0, y0, z0)$ of the position vector OO" at the position O" of the image position/orientation detection coil 31 and the directional components $(xi, yi, zi)$ of the position vector of each of the plurality of insertion shape detection coils 32 on the orthogonal coordinate axis O-xyz, and outputs the created insertion shape data to the synthesis circuit 58.

This situation is shown in FIG. 11. The insertion shape data is image data on the orthogonal coordinate axis O'-x'y'z' in which a string-like insertion shape marker Ms sequentially joining together the positions of the image position/ orientation detection coil 31 and the plurality of insertion shape detection coils 32 and then interpolating the positions is synthesized with a coil position marker Mc indicating the position of each of the coils.

(d) The flows of reference image data and data created by processing the reference image data

The operator instructs acquisition of reference image data by pressing a predetermined key on the keyboard 13 or selecting a menu on the screen by the mouse 12. At this time, the operator instructs an acquisition source at the same time. In response to the instruction, the control circuit 63 issues an instruction to the communication circuit 54 on the loading of the reference image data and the acquisition source of the data.

In a case where the acquisition source is the X-ray three-dimensional helical CT apparatus 15, for example, the communication circuit 54 loads a plurality of two-dimensional CT images as reference image data through the network 17 to store the data in the reference image storage portion 55. When the X-ray three-dimensional helical CT apparatus 15 is used to pick up images, a contrast agent is injected from blood vesselsof the subject 37 before image pickup so as to allow blood vessels (vascular channels in a broad sense) such as aorta and superior mesenteric vein and organs containing a large number of blood vessels to be displayed at a high or a medium luminance on the two-dimensional CT image, thereby easily differentiating the luminance from that of surrounding tissues.

In addition, in a case where the acquisition source is the three-dimensional MRI apparatus 16, for example, the communication circuit 54 loads a plurality of two-dimensional MRI images as reference image data through the network 17 to store the data in the reference image storage portion 55. When the three-dimensional MRI apparatus 16 is used to pick up images, an MRI contrast agent with a high nuclear magnetic resonance sensitivity is injected from blood vessels of the subject 37 before image pickup so as to allow blood vessels such as aorta and superior mesenteric vein and organs containing a large number of blood vessels to be displayed at a high or a medium luminance on the two-dimensional MRI image, thereby easily differentiating the luminance from that of the surrounding tissues.

Since the workings are the same in either case where the operator selects the X-ray three-dimensional helical CT apparatus 15 or the three-dimensional MRI apparatus 16 as data acquisition source, the working will be described only on the case where the operator selects the X-ray three-dimensional helical CT apparatus 15 and the communication circuit 54 loads a plurality of two-dimensional CT images as reference image data.

FIG. 5 shows an example of the reference image data stored in the reference image storage portion 55. Under the effect of the X-ray contrast agent, the blood vessels such as the aorta and the superior mesenteric vein are displayed at a high luminance, the organ such as the pancreas which contains a large number of peripheral arteries is displayed at a medium luminance, and the duodenum and the like are displayed at a low luminance.

The interpolation circuit 56 reads all the 1 st to N-th reference image data from the reference image storage portion 55. Next, the interpolation circuit 56 fills the read reference image data into a voxel space in the interpolation memory 56a. Specifically, the interpolation circuit 56 outputs the luminances of the pixels in the reference image data to the voxels having addresses corresponding to the pixels. Next, the interpolation circuit 56 performs interpolation on the basis of the luminance values of the adjacent reference image data to fill empty voxels with the data. Thus, all the voxels in the voxel space is filled with the data based on the reference image data (hereinafter referred to as voxel data).

The three-dimensional human body image creation circuit 57 extracts voxels of a high luminance value (mostly the blood vessels) and voxels of a medium luminance value (mostly the organ such as the pancreas which contains a large number of peripheral blood vessels) according to the luminance value range from the interpolation circuit 56, and classifies the voxels into each luminance to color the voxels. Next, the three-dimensional human body image creation circuit 57 fills the extracted voxels as the three-dimensional human body image data in the voxel space of the synthesis memory 58a in the synthesis circuit 58. At this time, the three-dimensional human body image creation circuit 57 fills the voxel spaces with the extracted voxels so that the address of each extracted voxel in the voxel space in the interpolation memory 56a is the same as that in the voxel space in the synthesis memory 58a.

FIG. 12 shows an example of the three-dimensional human body image data. In the example shown in FIG. 12, the three-dimensional human body image data extracts the aorta and the superior mesenteric vein which are the blood vessels at a high luminance, and the organ at a medium luminance, the pancreas. The blood vessels and the pancreas are colored in red and green, respectively, and the data is shown as the three-dimensional data of when the subject 37 is observed from the ventral side with the head side on the right, and the foot side on the left.

The synthesis circuit 58 fills image index data and insertion shape data into the voxel space in the synthesis memory 58a. The situation is shown in FIG. 13. In FIG. 13, for convenience of the description, the three-dimensional human body image data present in the voxel space is omitted (the case where the three-dimensional human body image data is not omitted is shown in FIG. 14 and the like). Thus, the synthesis circuit 58 fills the three-dimensional image data, the image index data, and the insertion shape data into the same voxel space, thereby synthesizing the data into one set of data (hereinafter referred to as synthetic three-dimensional data).

The three-dimensional guide image creation circuit A executes a rendering processing such as hidden surface removal or shading on the synthetic three-dimensional data to create image data (hereinafter referred to as three-dimensional guide image data) that can be outputted to the screen. The default direction of the three-dimensional guide image data is assumed to be the direction from the ventral side of the human body. Therefore, the three-dimensional guide image creation circuit A creates the three-dimensional image data of when the subject 37 is observed from the ventral side direction.

Note that, although the default direction of the three-dimensional guide image data is the direction from the ventral side of the human body, the three-dimensional guide image data of when the subject is observed from the dorsal side direction may be created. In addition, the three-dimensional guide image data of when the subject is observed from other directions may be created.

Next, the three-dimensional guide image creation circuit A outputs the three-dimensional guide image data of when the subject is observed from the ventral side to the mixing circuit 61. The three-dimensional guide image data is shown in FIG. 14. The right side of FIG. 14 is the head side of the subject and the left side is the foot side of the subject. In the three-dimensional guide image data in FIG. 14, the ultrasound tomographic image marker Mu, contained in the image index data, is translucent so that the 6 o'clock direction marker Mt and distal direction marker Md, contained in the image index data, and the insertion shape marker Ms and coil position marker Mc, contained in the insertion shape data, can be seen through. The ultrasound tomographic image marker Mu is opaque with respect to viscera so as to make invisible the viscera hidden by the ultrasound tomographic image marker Mu. In FIG. 14, each of the markers located behind and overlapping the ultrasound tomographic image marker Mu are shown by dashed lines.

The rotational transformation circuit 59 reads the synthetic three-dimensional data to perform rotational processing on the read synthetic three-dimensional data according to a rotation instruction signal from the control circuit 63.

The three-dimensional guide image creation circuit B executes a rendering processing such as hidden surface removal or shading on the synthetic three-dimensional data subjected to the rotational processing to create image data that can be outputted to the screen. In the present embodiment, as an example, it is assumed that the operator gives an instruction to observe the three-dimensional guide image data with a line of sight along the normal line of the ultrasound tomographic image marker Mu by the input through the mouse 12 or the keyboard 13. That is, the rotation instruction signal outputted from the control circuit 63 instructs to observe the three-dimensional guide image data with the line of sight oriented in the - V direction, based on the position/orientation mapping data. The rotational transformation circuit 59 creates guide images on which the normal line of the ultrasound tomographic image marker Mu on the synthetic three-dimensional data is set so as to coincide with the observation line of sight, that is, the normal line of the screen of the display apparatus 14 and the six o'clock direction marker Mt is set so as to orient downward on the screen of the display apparatus 14.

In addition, as shown in FIG. 15, the three-dimensional guide image creation circuit B creates three-dimensional guide image data such that the ultrasound tomographic image marker Mu among the image index data is set to be translucent, and not only the 6 o'clock direction marker Mt and distal end direction marker Md on the image index data and the insertion shape marker Ms and coil position marker Mc on the insertion shape data but also those parts of the other viscera which are located behind the ultrasound tomographic image marker Mu can be seen through.

Specifically, the three-dimensional guide image creation circuit B creates three-dimensional guide image data such that the part on the ultrasound tomographic image marker Mu is displayed in dark color, the part located in front of the ultrasound tomogaphic image marker Mu is not displayed, and the part located behind the ultrasound tomographic image marker Mu is displayed at a lower luminance. Then the three-dimensional guide image creation circuit B outputs the created three-dimensional guide image data to the mixing circuit 61. As for the pancreas, the three-dimensional guide image data is created such that the part on the ultrasound tomographic image marker Mu is displayed in dark green, and the part behind the marker Mu is displayed in light green. As for blood vessels, the three-dimensional guide image data is created such that the part on the ultrasound tomographic image marker Mu is displayed in dark red and the part behind the marker Mu is displayed in light red.

The three-dimensional guide image creation circuit B outputs the three-dimensional guide image data thus created to the mixing circuit 61.

(e) The flows of signals and data related to a final display screen obtained by synthesizing ultrasound tomographic image data with three-dimensional guide image data

The mixing circuit 61 in FIG. 4 creates mixing data for adjacently displaying the ultrasound tomographic image data from the ultrasound observation apparatus 4, the three-dimensional guide image data of when the subject 37 is observed from the ventral side, which is from the three-dimensional guide image creation circuit A, and the three-dimensional guide image data of when the subject 37 is observed from the same direction as that of the ultrasound tomographic image, which is from the three-dimensional guide image creation circuit B.

The display circuit 62 converts the mixing data into an analog video signal to output to the display apparatus 14. The display apparatus 14, based on the analog video signal, adjacently displays the utlrasound tomographic image, the three-dimensional guide image of when the subject 37 is observed from the ventral side, and the three-dimensional guide image of when the subject 37 is observed from the same direction as that of the ultrasound tomographic image.

As shown in FIG. 16, the display apparatus 14 displays the organs expressed on the three-dimensional guide image in the respective colors corresponding to the original luminance values on the reference image data. In the display example in FIG. 16, the pancreas is displayed in green, and the aorta and the superior mesenteric vein are displayed in red. In FIG. 16, the markers located behind and overlapping the ultrasound tomographic image marker Mu are shown by dashed lines. Here, the three-dimensional guide image of when the subject is observed from the ventral side is substantially a wide range guide image, and the three-dimensional guide image of when the subject is observed from the same direction as that of the ultrasound tomographic image is a detailed guide image.

(f) The flows of signals and data related to control

All the following components in the image processing apparatus 11 of FIG. 4 are controlled based on the instruction from the control circuit 63: the matching circuit 51; the image index creation circuit 52; the insertion shape creation circuit 53; the communication circuit 54; the reference image storage portion 55; the interpolation circuit 56, the three-dimensional human body image creation circuit 57; the synthesis circuit 58, the rotational transformation circuit 59; three-dimensional guide image creation circuit A, the three-dimensional guide image creation circuit B, the mixing circuit 61, and the display circuit 62. Details of the control will be described later.

Hereinafter, the whole working of the image processing apparatus 11, the keyboard 13, the mouse 12, and the display apparatus 14 of the present embodiment will be described according to the operator's usage pattern. FIG. 17 is a whole flowchart, and the respective processings in steps S1 to S4 are executed in the order shown in the figure.

The first step S1 is a specification processing of the body surface feature points and the body cavity feature point on reference image data. That is, in the step S1, the processing of specifying the body surface feature points and the body cavity feature point on reference image data is performed.

In the next step S2, the operator fixes the body surface detection coils 7 to the subject 37. The operator makes the subject 37 lie on his or her left side, that is, lie in the so-called left lateral position. The operator palpates the subject 37 and fixes the body surface detection coils 7 to the positions on the body surface which are closest to the four body surface feature points, that is, the xiphoid process, the left anterior superior iliac spine, the right anterior superior iliac spine, and the spinous process of vertebral body.

The next step S3 is a correction value calculation processing. In the step S3, the image processing apparatus 11 obtains position/orientation data of the body cavity feature point to calculate a conversion equation that maps position/orientation data expressed on the orthogonal coordinate axis O-xyz into position/orientation mapping data in the voxel space expressed on the orthogonal coordinate axis O'-x'y'z'. The image processing device 11 further calculates a correction value for the conversion equation based on the body cavity feature point coordinates.

In the next step S4, ultrasound tomographic image and three-dimensional guide image creation/display processing is performed. The step S4 is the processing of creating and displaying ultrasound tomographic images and three-dimensional guide images.

Next, specific description will be made on the processing in step S 1 in the flowchart of FIG. 17, that is, the body surface feature points and the body cavity feature point specification processing on the reference image data. FIG. 18 shows a detail of the processing of specifying body surface feature points and body cavity feature point on the reference image data in step S 1 of FIG. 17.

In the first step S1-1, the operator presses the display switching key 13α. The control circuit 63 issues an instruction to the display circuit 62. In response to the instruction, the switch 62a of the display circuit 62 is switched to the input terminal α.

In the next step S1-2, the operator specifies any of the 1 st to N-th reference image data using the mouse 12 and keyboard 13.

In the next step S1-3, the control circuit 63 causes the display circuit 62 to read specified reference image data among the 1st to N-th reference image data stored in the reference image storage portion 55. The display circuit 62 converts the reference image data from the reference image storage portion 55 into an analog video signal to output the reference image data to the display apparatus 14. The display apparatus 14 displays the reference image data.

In the next step S1-4, the operator specifies the body surface feature points on the reference image data through the mouse 12 and keyboard 13. The specific procedure is described as follows.

The operator performs an operation such that the displayed reference image data contains any of the four body surface feature points of the subject 37, that is, the xiphoid process, the left anterior superior iliac spine, the right anterior superior iliac spine, and the spinous process of vertebral body. If the reference image data contains none of the above body surface feature points, the processing returns to step S1-2, where the operator respecifies another reference image data, and in step S1-3, repeatedly display different reference image data until the reference image data containing any of the feature points is displayed.

The operator specifies on the displayed reference image data, using the mouse 12 and the keyboard 13, pixels corresponding to points on the body surface of the subject 37 which are closest to the four points on the body surface, that is, the xiphoid process, the left anterior superior iliac spine, the right anterior superior iliac spine, and the spinous process of vertebral body. The specified points are shown by black circles • and white circles o in FIG. 9.

In the present embodiment, for the convenience of description, description is made assuming that the xiphoid process o is contained in the n-1th reference image data ($1 \leq nl \leq N$), and the left anterior superior iliac spine, the right anterior superior iliac spine, and spinous process of vertebral body • are contained in the n-2th reference image data ($1 \leq n2 \leq N$). In FIG. 9, for the convenience of description, the xiphoid process is shown by the white circle o at the position corresponding to the xiphoid process on the n-2th reference image data.

In the next step S1-5, the operator specifies the body cavity feature point P" using the mouse 12 and keyboard 13. In the present embodiment, description will be made taking the duodenal papilla (opening of the common bile duct into the duodenum) as an example of body cavity feature point P". The specific procedure is described as follows.

The operator specifies any of the 1st to N-th reference image data using the mouse 12 and the keyboard 13. The control circuit 63 causes the display circuit 62 to read the specified reference image data among the reference image data 1st to N-th stored in the reference image storage portion 55 via a signal line not shown. The display circuit 62 outputs the read reference image data to the display apparatus 14. The display apparatus 14 displays the reference image data. If the duodenal papilla as the body cavity feature point of the subject 37 is not contained in the reference image data, the operator respecifies another reference image data and repeatedly displays different reference image data until the reference image data containing the duodenal papilla is displayed.

The operator specifies the pixels corresponding to the duodenal papilla as the point in the body cavity of the subject 37 on the displayed reference image data, using the mouse 12 and the keyboard 13. The specified point is shown by P" in FIG. 9. In the present embodiment, for the convenience of description, description will be made assuming that the duodenal papilla P" is contained in the n2-th reference image data ($1 \leq n2 \leq N$).

In the next step S 1-6, the control circuit 63 calculates the coordinates, on the orthogonal coordinate axis O'-x'y'z' in the voxel space, of the respective pixels corresponding to the body surface feature points specified in step S1-4 and of the pixels corresponding to the body cavity feature point P" specified in step S1-5, based on the addresses on the reference image data. The control circuit 63 then outputs the coordinates to the matching circuit 51.

The calculated values of the coordinates, on the orthogonal coordinate axis O'-x'y'z', of the respective pixels corresponding to the body surface feature points specified in step S1-4 are defined as (xa', ya', za'), (xb', yb', zb'), (xc', yc', zc'), and (xd', yd', zd'). The calculated value of the coordinates, on the orthogonal coordinate axis O'-x'y'z', of the pixels corresponding to the body cavity feature point specified in step S1-5 are defined as (xp", yp", zp"). The matching circuit 51 stores the coordinates.

After the step S1-6 ends, the processing proceeds to the step S2 in FIG. 17. After the processing in step S2, the processing proceeds to the correction value calculation processing in the step S3 in FIG. 17. Detail of the correction value calculation processing in step S3 is displayed in FIG. 19.

As described above, the step S3 is the processing of obtaining position/orientation data of body cavity feature point to calculate a conversion equation that maps position/orientation data expressed on the orthogonal coordinate axis O-xyz into position/orientation mapping data in the voxel space expressed on the orthogonal coordinate axis O'-x'y'z', and then calculating correction value for the conversion equation from the position/orientation data of the body cavity feature point.

When the correction value calculation processing in step S3 in FIG. 17 is started, the operator presses the display switching key 13β in the first step S3-1 in FIG. 19. In response to this instruction, the control circuit 63 issues an instruction to the display circuit 62. The switch 62a of the display circuit 62 is switched to the input terminal β according to the instruction.

Next, in step S3-2, the display circuit 62 converts optical image data from the optical observation apparatus 3 into an analog video signal to output the optical image to the display apparatus 14. The display apparatus 14 displays the optical image.

In the next step S3-3, the operator inserts the rigid portion 21 and the flexible portion 22 of the ultrasound endoscope 2 into the body cavity of the subject 37.

In the next step S3-4, the operator moves the rigid portion 21 to search for the body cavity feature point, while observing the optical image. After the body cavity feature point was found, the operator moves the rigid portion 21 in the vicinity of the body cavity feature point.

In the next step S3-5, the operator inserts the body cavity contact probe 8 from the forceps port 44 to project the body cavity contact probe 8 from the projection port 45 while observing the optical image. Then, the operator brings the distal end of the body cavity contact probe 8 into contact with the body cavity feature point under the optical image field of view. This situation is shown in FIG. 20. In FIG. 20, an optical image is displayed on the display screen. The duodenal papilla P as an example of the body cavity feature point and the body cavity contact probe 8 are displayed on the optical image.

In the next step S3-6, the operator presses the body cavity feature point specification key 65. In the next step S3-7, the control circuit 63 issues an instruction to the matching circuit 51. In response to the instruction, the matching circuit 51 loads the position/orientation data from the position/orientation calculation apparatus 5 to store the data.

As described above, the position/orientation data includes two types of data: the directional components of each of the position vectors of the four body surface detection coils 7 on the orthogonal coordinate axis O-xyz, that is, in this case, the coordinates of the four body surface feature points on the orthogonal coordinate axis O-xyz, (xa, ya, za), (xb, yb, zb), (xc, yc, zc), and (xd, yd, zd); and the directional components of each of the position vectors of the body cavity detection coil 42 on the orthogonal coordinate axis O-xyz, that is, in this case, the coordinates of the body cavity feature point on the orthogonal coordinate axis O-xyz, (xp, yp, zp).

In the next step S3-8, the matching circuit 51 creates a first conversion equation expressing a first mapping, from the coordinates of the body surface feature points. The specific procedure is as follows.

First, the matching circuit 51 already stores the following contents.

First: the coordinates, on the orthogonal coordinate axis O'-x'y'z' in the voxel space, of the pixels corresponding to the body surface feature points specified in step S 1, (xa', ya', za'), (xb', yb', zb'), (xc', yc', zc'), and (xd', yd', zd').

Second: the coordinates, on the orthogonal coordinate axis O'-x'y'z' in the voxel space, of the pixels corresponding to the body cavity feature point specified in step S1, (xp", yp", zp").

Third: the coordinates, on the orthogonal coordinate axis O-xyz, of the body surface feature points loaded in step S3-7, (xa, ya, za), (xb, yb, zb), (xc, yc, zc), and (xd, yd, zd).

Fourth: the coordinates, on the orthogonal coordinate axis O-xyz, of the body cavity feature point loaded in step S3-7, (xp, yp, zp).

The matching circuit 51 creates a first conversion equation that expresses the first mapping from an arbitrary point on the orthogonal coordinate axis O-xyz to an appropriate point on the orthogonal coordinate axis O'-x'y'z' in the voxel space, from the third coordinates (xa, ya, za), (xb, yb, zb), (xc, yc, zc), and (xd, yd, zd) and the first coordinates (xa', ya', za'), (xb', yb', zb'), (xc', yc', zc'), and (xd', yd', zd'). The first mapping and the first conversion equation are defined as follows.

As shown in FIG. 9, the xiphoid process, the left anterior superior iliac spine, the right anterior superior iliac spine, and the spinous process of vertebral body which are the body surface feature points, are used to assume (set) two oblique coordinate systems with three vectors extending from the xiphoid process to the other points as fundamental vectors, on the subject 37 and in the voxel space (though the voxel space is expressed as reference image data in FIG 9, the voxel space is actually a data space obtained by interpolating the reference image data).

The first mapping means a mapping from the subject 37 to the voxel space such that "the coordinates of an arbitrary point on the orthogonal coordinate axis O-xyz expressed by the oblique coordinate system on the subject 37" are the same as "the coordinates of the point resulting from the mapping of the arbitrary point on the orthogonal coordinate axis O'-x'y'z' expressed by the oblique coordinate system in the voxel space". Furthermore, the first conversion equation means an equation for converting "the coordinates on the arbitrary point on the orthogonal coordinate axis O-xyz" into "the coordinates on the point resulting from the first mapping on the orthogonal coordinate axis O'-x'y'z' in the voxel space ".

For example, as shown in FIG. 9, the point resulting from the first mapping of the position of the image position/ orientation detection coil 31, that is, the center of the radial scan and the center of ultrasound tomographic image O" is assumed to be Q'. The coordinates of the point Q' on the orthogonal coordinate axis O'-x'y'z' are assumed to be (x0', y0', z0'). When using the first conversion equation, the coordinates (x0, y0, z0) of the point O" on the orthogonal coordinate axis O-xyz are converted into the coordinates (x0', y0', z0') of the point Q' on the orthogonal coordinate axis O'-x'y'z'.

In the next step S3-9, the matching circuit 51 maps the body cavity feature point P to the point P' in the voxel space using the first conversion equation, as shown in FIG. 9. The coordinates of the body cavity feature point P on the orthogonal coordinate axis O-xyz are (xp, yp, zp). The coordinates of the point P' resulting from the first mapping on the orthogonal coordinate axis O'-x'y'z' are defined as (xp', yp', zp').

In the next step S3-10, the matching circuit 51 calculates a vector P'P" as follows from the coordinates (xp', yp', zp') of the point P' on the orthogonal coordinate axis O'-x'y'z' in the voxel space and the coordinates (xp", yp", zp") of the point P"corresponding to the body cavity feature point specified in step S1, on the orthogonal coordinate axis O'-x'y'z' in the voxel space,

$$P'P'' = (xp'', yp'', zp'') - (xp', yp', zp') = (xp''\text{-}xp', yp''\text{-}yp', zp''\text{-}zp')$$

In the next step S3-11, the matching circuit 51 stores the vector P'P". The vector P'P" woks as a correction value for correcting the first conversion equation to create a second conversion equation in the processing to be described later. After the step S3-11 ends, the processing proceeds to the next step S4.

Next, description will be made on the ultrasound tomographic image and three-dimensional guide image creation/display processing in step S4, with reference to FIG. 21. FIG. 21 is a detail of the processing of creating and displaying actual ultrasound tomographic images and the three-dimensional guide images of the subject 37 in step S4.

When the processing in step S4 is started, the operator presses the display switching key 13γ in the first step S4-1. The control circuit 63 issues an instruction to the display circuit 62. In response to the instruction, the switch 62a of the display circuit 62 is switched to the input terminal γ.

In the next step S4-2, the operator presses the scan control key 66. In the next step S4-3, the control circuit 63 outputs a scan control signal to the ultrasound observation apparatus 4. Then, the ultrasound transducer array 29 starts radial scan.

In the next step S4-4, the control circuit 63 issues an instruction to the mixing circuit 61. In response to the instruction, the mixing circuit 61 sequentially loads ultrasound tomographic image data inputted from the ultrasound observation apparatus 4 in response to the radial scanning.

In the next step S4-5, the control circuit 63 issues an instruction to the matching circuit 51. In response to the instruction, the matching circuit 51 loads the position/orientation data from the position/orientation calculation apparatus 5 and stores the loaded data. The loading is instantaneously performed. Therefore, the matching circuit 51 loads the position/orientation data including the following data obtained at the moment when the mixing circuit 61 loads the ultrasound tomographic image data in step S4-4.

The directional components of the position vector OO" of O", which is the position of the image position/ orientation detection coil 31, that is the center of radial scan and the center of the ultrasound tomographic image, on the orthogonal coordinate axis O-xyz: (x0, y0, z0).

The angular components of the Euler angle indicating the orientation of the image position/orientation detection coil 31, that is, the orientation of the ultrasound tomographic image, with respect to the orthogonal coordinate axis O-xyz: ($\psi$, $\theta$, $\phi$).

The respective directional components of the position vectors of each of the plurality of insertion shape detection coils 32 on the orthogonal coordinate axis O-xyz: (xi, yi, zi) (i is a natural number between 1 and the total number of the insertion shape detection coils 32)

The respective directional components of the four body surface detection coils 7 on the orthogonal coordinate axis O-xyz: (xa, ya, za), (xb, yb, zb), (xc, yc, zc), and (xd, yd, zd).

In the next step S4-6, the matching circuit 51 updates the first conversion equation stored in the step S3 using the respective directional components of the four body surface detection coils 7 on the orthogonal coordinate axis O-xyz (xa, ya, za), (xb, yb, zb), (xc, yc, zc) and (xd, yd, zd) among the position/orientation data loaded in the step S4-5.

Next, the matching circuit 51 combines the updated first conversion equation with the translation by the vector P'P" stored in step S3 to create a new second conversion equation that expresses second mapping. The concept of the second mapping is as follows.

The second mapping = the first mapping + translation by the vector P'P" The translation by the vector P'P" has a correction effect shown below. The vector P'P" works as a correction value. The first mapping is the mapping from the subject 37 to the voxel space such that "the coordinates of an arbitrary point on the orthogonal coordinate axis O-xyz expressed by the oblique coordinate system on the subject 37" are the same as "the coordinates of the point resulting from the mapping of the arbitrary point on the orthogonal coordinate axis O'-x'y'z' expressed by the oblique coordinate system in the voxel space".

Ideally, the mapping point P' obtained by the first mapping of the body cavity feature point P into the voxel space preferably coincides with the point P" corresponding to the body cavity feature point specified in step S1. However, it is actually difficult to accurately coincide these points with each other.

The reason is that "the spatial positional relationship between an arbitrary point on the orthogonal coordinate axis O-xyz and the oblique coordinate system on the subject 37" and "the spatial positional relationship between the point on the orthogonal coordinate axis O'-x'y'z' anatomically corresponding to the arbitrary point and the oblique coordinate system in the voxel space" do not completely coincide with each other due to various factors. This is because, in the present embodiment, the first mapping and the first conversion equation are calculated from the respective coordinates of the body surface feature points, which are characteristic points on the skeleton, however, the duodenal papilla P as the body cavity feature point does not always have the same positional relationship with the body surface feature points on the skeleton.

The main cause is the displacement of the various organs in the subject 37 due to the effect of the gravity, because images are normally picked up with a patient being in a supine position when using the X-ray three-dimensional helical CT apparatus 15 and the three-dimensional MRI apparatus 16 and the body position of the patient is different from the left lateral position in the inspection using the ultrasound endoscope 2.

Therefore, the second mapping is the combination of the first mapping and the translation with the vector P'P" as a correction value, so that the mapping point of the body cavity feature point P coincides with the point P" corresponding to the body cavity feature point in the voxel space. Moreover, another point on the subject 37, for example, the center of the ultrasound tomographic image 0" also anatomically coincides with the body cavity feature point more accurately by the second mapping.

In the next step S4-7, the matching circuit 51 uses the newly created second conversion equation to convert, among the position/orientation data loaded in step S4-5, the directional components (x0, y0, z0) of the position vector OO" of the center of the ultrasound tomographic image O" on the orthogonal coordinate axis O-xyz, the angular components $(\psi, \theta, \phi)$ of the Euler angle indicating the orientation of the image position/orientation detection coil 31 with respect to the orthogonal coordinate axis O-xyz, and the directional components (xi, yi, zi) of the position vectors of each of the plurality of insertion shape detection coils 32, on the orthogonal coordinate axis O-xyz (i is a natural number between 1 and the total number of the insertion shape detection coils 32), into position/orientation mapping data.

As shown in FIG. 9, although the center of the ultrasound tomographic image O" is mapped to the point Q' on the voxel space by the first conversion equation, the center of the ultrasound tomographic image O" is mapped to the point Q" on the voxel space by the second conversion equation newly created in the present step, as shown in FIG. 9. The vector Q'Q" indicating the difference between Q' and Q" coincides with the correction by the translation in the second mapping, so that the vector Q'Q" is the same as the vector P'P". That is, the following equation is established.

$$Q'Q'' = P'P''$$

The next step S4-8 is the processing of creating three-dimensional guide image data. That is, the image index creation circuit 52 creates image index data. The insertion shape creation circuit 53 creates insertion shape data. The synthesis circuit 58 synthesizes three-dimensional human body image data, image index data, and insertion shape data, to create synthetic three-dimensional data. The rotational transformation circuit 59 executes a rotation processing on synthetic three-dimensional data. Each of the three-dimensional guide image creation circuits A and B creates three-dimensional guide image data. Each of the processings is as described above.

In the next step S4-9, the mixing circuit 61 creates mixing data for adjacently displaying the ultrasound tomographic image data and the three-dimensional guide image data. The display circuit 62 converts the mixing data into an analog video signal. Based on the analog video signal, the display apparatus 14 adjacently displays, as shown in FIG 16, the ultrasound tomographic image, the three-dimensional guide image of when the subject 37 is observed from the ventral side, and the three-dimensional guide image of when the subject 37 is observed from the same direction as that of the ultrasound tomographic image. Each of the processings is as described above.

In the next step S4-10, the control circuit 63 confirms whether or not the operator presses the scan control key 66 again during steps S4-4 to S4-9. If the operator has pressed the scan control key 66 again, the control circuit 63 terminates the above processing here and outputs a scan control signal to the ultrasound observation apparatus 4 to instruct the radial scan control to be turned off. This causes the ultrasound transducer array 29 to terminate the radial scan. If the operator has not pressed the scan control key 66 again, the processing jumps to step S4-4.

The processings described in steps S4-4 to S4-9 are thus repeated, and thereby the ultrasound transducer array 29 performs one radial scan, the ultrasound observation apparatus 4 creates the ultrasound tomographic image data, and every time the ultrasound tomographic image data is inputted from the ultrasound observation apparatus 4 to the mixing circuit 61, two new three-dimensional guide images are created to be displayed together with the new ultrasound tomographic image on the display screen of the display apparatus 14 in real time, while being updated.

That is, as shown in FIG. 16, the ultrasound tomographic image marker Mu, the distal direction marker Md, and the six o'clock direction marker Mt on the image index data and the insertion shape marker Ms and the coil position marker Mc on the insertion shape data are moved or deformed on the three-dimensional human body image data in conjunction with movement of the radial scanning surface associated with the operator's manual operation of the flexible portion 22 and the rigid portion 21.

The above is the working until the three-dimensional guide image is displayed. In the present embodiment, the following working is added before the above working.

The reference image storage portion 55 previously stores reference image data for a plurality of body positions which have been obtained with the human body of the subject 37 himself or herself, or of a person other than the subject 37 set in a plurality of different body positions. In the present embodiment, the reference image storage portion 55 stores supine position reference image data obtained by the X-ray three-dimensional helical CT apparatus 15 or the three-dimensional MRI apparatus 16 with the body position of the subject set in the supine position, and left lateral position reference image data obtained by the X-ray three-dimensional helical CT apparatus 15 or the three-dimensional MRI apparatus 16 with the body position of the subject set in the left lateral position.

FIGS. 22, 23 respectively show examples of the supine position reference image data and the left lateral position reference image data. FIGS. 22, 23 respectively show the supine position reference image data and the left lateral position reference image data in which the positions in body axis direction of the human body are the same, in other words, the tomographic images whose z' axis coordinates are the same, seen from the foot side. In FIGS. 22, 23, the arrow G direction is the direction of gravitational force, that is, the vertical downward direction when each of the supine position reference image data and the left lateral position reference image data is obtained.

In FIGS. 22, 23, only the aorta, the inferior vena cava, the portal vein, the pancreas, and the gallbladder are extracted and shown. As shown in FIGS. 22, 23, it is understood that the positions of the organs are different for each body position when comparing the positions of the organs in the supine position (FIG. 22) and in the left lateral position (FIG. 23). This is because the directions of the gravitational force acting on each of the organs are different in the different body positions. Specifically, on the contrary to the supine position reference image data (FIG. 22) based on the image picked up in the supine position, each of the organs is displaced due to the effect of the gravitational force so as to rotate in the clockwise direction seen from the foot side in the left lateral position reference image data (FIG. 23) based on the image picked up in the left lateral position.

Then, in the present embodiment, the selection between the supine position reference image data and the left lateral position reference image data is performed using the keyboard 13 and the mouse 12 as state selection portions, and the control circuit 63. For example, every time the operator depresses a body position selection key 67 on the keyboard 13, the supine position reference image data or the left lateral position reference image data is alternatively selected.

The operator selects the reference image data obtained in the same body position as that of the subject 37. Specifically, the body position of the subject 37 in a case of diagnosis using the ultrasound endoscope 2 according to the present embodiment is generally in the left lateral position, so that the operator selects the left lateral position reference image data as the reference image data. Then, based on the selected reference image data obtained in the left lateral position, the three-dimensional human body image data is created as described above, and the guide image is displayed on the display apparatus 14.

The medical guiding system 1 according to the present embodiment having the above configuration creates a guide image to display the position and orientation of the ultrasound endoscope 2 as a medical instrument on the previously obtained reference image data, and as the reference image data, in particular, the left lateral position reference image data obtained in the left lateral position, which is the body position of the subject 37 when using the ultrasound endoscope 2, is used.

Therefore, in the present embodiment, the direction of the gravitational force acting on the viscera and the organs of the subject in which the ultrasound endoscope 2 as a medical instrument is used coincides with the direction of the gravitational force acting on the viscera and the organs of the human body from which the reference image data is obtained. Therefore, the present embodiment enables the anatomical positions of the viscera and the organs on the reference image data to accurately coincide with the anatomical positions of the viscera and the organs of the subject, so that regardless of the body position of the subject, the position of the medical instrument on the guide image can be coincided with the actual position of the medical instrument with high accuracy.

In addition, in the medical guiding system 1 of the present embodiment, the reference image storage portion 55 as storage means stores the supine position reference image data and the left lateral position reference image data as a plurality of reference image data by each body position which are obtained in different body positions. Then, the operator operates the body position selection key 67 on the keyboard 13 as body position information obtaining means, and thereby the reference image data obtained in generally the same body position as that of the subject 37 is used as the reference image data in creating the guide image.

With such a configuration according to the present embodiment, even if a plurality of options exist in the body positions of the subject when using a medical instrument, the direction of the gravitational force acting on the viscera and the organs of the subject can be coincided with the direction of the gravitational force acting on the viscera and the organs of the human body from which the reference image data is obtained, in each body position. Therefore, regardless of the body position of the subject, the anatomical positions of the viscera and the organs on the reference image data can be accurately coincided with the anatomical positions of the viscera and the organs of the subject, thereby enabling the position of the medical instrument on the guide image to coincide with the actual position of the medical instrument with high accuracy.

Note that, in the present embodiment, the body position of the subject 37 is selected by the operator's operation of the keyboard 13, however the present invention is not limited to the configuration. For example, the body position of the subject 37 may be calculated from a relative positional relationship on the orthogonal coordinate axis O-xyz of the plurality of body surface detection coils 7 mounted to the subject 37, to automatically select the reference image data obtained in generally the same body position as the calculated body position.

Furthermore, in the present embodiment, although description was made on the reference image data obtained in the supine position and the left lateral position, the reference image data is obtained in accordance with the body position at the time of using the medical instrument, so that it is needless to say that the reference image data may be obtained in other body positions such as prone position, for example.

Note that, in the above-described present embodiment, the medical guiding system 1 includes the ultrasound endoscope 2 including the treatment instrument channel 46, and the body cavity contact probe 8 inserted through the treatment instrument channel 46, however the configuration is not limited to this.

If the objective lens 25 focuses on the body cavity feature point via the optical observation window 24, and the rigid portion 21 itself can be brought accurately into contact with the body cavity feature point without using the body cavity contact probe 8, the image position/orientation detection coil 31 fixedly provided to the rigid portion 21 may be a substitute for the body cavity detection coil 42 of the body cavity contact probe 8. At this time, the image position/ orientation detection coil 31 also serves not only as the image position/orientation detection element but also as the body cavity detection element.

Furthermore, in the present embodiment, electronic radial scan ultrasound endoscope 2 is used as the ultrasound probe, however a mechanical scan ultrasound endoscope, an electronic convex scan ultrasound endoscope including a group of ultrasound transducers provided in one of the insertion axis in a fan shape, or capsule ultrasound sonde may be used, and there is no limitation placed on the ultrasound scanning method. Furthermore, an ultrasound probe without the optical observation window 24 may be used.

Furthermore, in the present embodiment, in the rigid portion 21 of the ultrasound endoscope 2, the ultrasound transducers cut into small pieces like strips are arranged around the insertion axis as an annular array, however, the ultrasound transducer array 29 may be provided all around the circumference of the insertion axis through 360 degrees or may be provided around the circumference through less than 360 degrees. For example, the ultrasound transducer array 29 may be formed around the circumference of the rigid portion 21 through 270 degrees or 180 degrees.

Moreover, in the present embodiment, the transmission antenna 6 and the reception coil are used as position detection means to detect the position and orientation based on the magnetic fields, however the transmission and reception relationship may be reversed. When the position and orientation are detected using the magnetic fields, the position (orientation) detection means can be formed with a simple configuration as well as with reduced cost and size. However the detection method is not limited to one using the magnetic fields, and position and orientation may be detected on the basis of acceleration or other means. Furthermore, the present embodiment sets the origin O at the particular position on the transmission antenna 6. However, the origin O may be set in another position having the same positional relationship with respect to the transmission antenna 6.

Furthermore, in the present embodiment, the image position/orientation detection coil 31 is fixedly provided to the rigid portion 21. However, the image position/orientation detection coil 31 needs not be provided entirely inside the rigid portion 21 as far as the position of the image position/orientation detection coil 31 is fixed with respect to the rigid portion 21. In addition, in the present embodiment, the organs on the three-dimensional guide image data are displayed in different colors by each organ. However, the present invention is not limited to the use of the variation in display color but may use another aspect such as luminance, brightness, color saturation, or the like. For example, luminance values may be changed by each organ.

Moreover, in the present embodiment, a plurality of two-dimensional CT images or the two-dimensional MRI images picked up by the X-ray three-dimensional helical CT apparatus 15 or the three-dimensional MRI apparatus 16 are used as the reference image data. However, three-dimensional image data previously obtained using another modality such as PET (Positoron Emission Tomography) may be used. Alternatively, the three-dimensional image data previously obtained by a so-called extracoporeal ultrasound diagnostic apparatus, which employs a method of irradiating ultrasound from outside the body, may be used.

In addition, the present embodiment includes the body surface detection coils 7 composed of four coils wound in one axis direction, and the coils are detachably fixed to the plurality of body surface feature points on the subject's body surface with a tape, belt, band or the like, to simultaneously obtain position/orientation data of the body surface feature points. However, instead of using one coil, for example, the body cavity detection coil 42, the subject 37 is made to be in the left lateral position prior to the inspection using the ultrasound endoscope 2, to sequentially obtain the position/orientation data of the body surface feature points by bringing the distal end of the body cavity contact probe 8 into sequentially contact with the plurality of body surface feature points.

Furthermore, in the present embodiment, the position/orientation calculation apparatus calculates the positions with respect to the body surface detection coils 7 as position/orientation data. However, the direction of the winding axis of the body surface detection coils 7 may be calculated instead of the positions thereof, or both of the positions and the direction of the winding axis may be calculated. The increased degree of freedom for calculation by the position/orientation calculation apparatus 5 with respect to one of the body surface detection coils 7 can reduce the number of the body surface detection coils 7, and thus can reduce the burden imposed on the operator and the subject 37 when the body surface detection coils 7 are fixed to the subject 37 or during the ultrasound endoscopy.

Furthermore, in the present embodiment, description has been made on the body surface feature points as the points of the abdominal body surface, that is, the xiphoid process, the left anterior superior iliac spine, the right anterior superior iliac spine, and spinous process of vertebral body, and the body cavity feature point as the duodenal papilla. However, the present invention is not limited to the example. The feature points may be located on the body surface of the chest or in a chest cavity, or another example may be employed. Generally, the orientation of the ultrasound tomographic image marker Mu is more accurately determined when the body surface feature points are taken on the points associated with the skeleton.

(Second Embodiment)

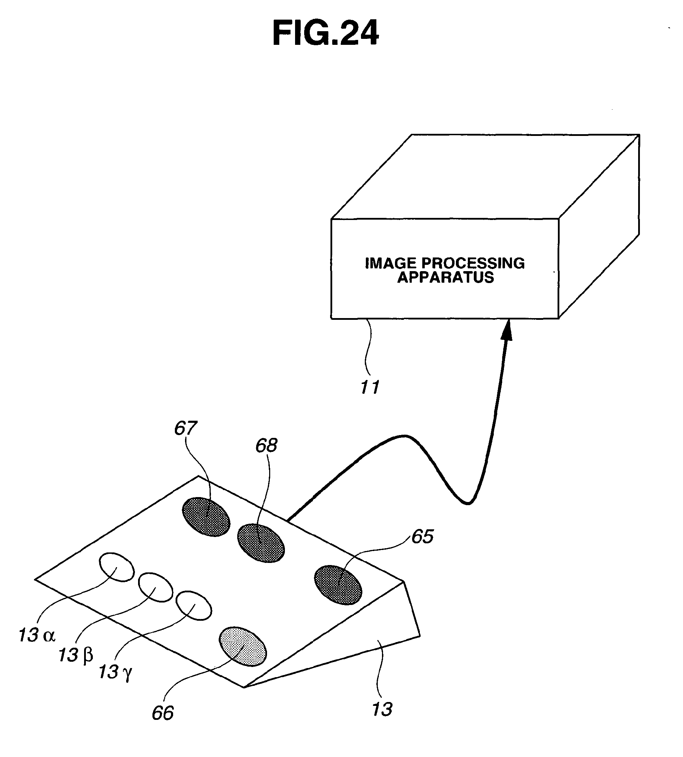

The second embodiment of the present invention is described below. The present embodiment differs in only a part of components and actions from the first embodiment. Therefore, only the different points are described below. In addition, the same components as those in the first embodiment are attached with the same reference numerals and the description thereof will be omitted appropriately. FIG. 24 is an illustration diagram showing a key arrangement of a keyboard.

In the present embodiment, the reference image storage portion 55 stores not only the plurality of reference image data by body position described in the first embodiment but also the plurality of reference image data by feature obtained according to physical features of a human body.

Here, the physical features of the human body indicate at least one of physical size, body height, sex, age, anamnesis, surgical history, and the like, and the plurality of reference image data by feature are stored in the reference image storage portion 55, as the reference image data by feature, with the physical feature information added. The reference image data by feature are, after setting a plurality of conditions for at least one item of physical size, body height, sex, age, anamnesis and surgical history, obtained in advance from the human body satisfying the conditions.

Note that, these physical feature data and the reference image data by feature may be stored in the reference image storage portion as separate data, and the physical features and the plurality of reference image data by feature may be associated with each other in a database not shown.

Furthermore, the keyboard 13 according to the present embodiment, as shown in FIG. 24, further includes a physical feature selection key 68 to input physical features of the subject, in addition to the body position selection key 67 for the operator to input the body position of the subject.

The operator's operation of the physical feature selection key on the keyboard 13 allows the reference image data by feature obtained in the condition coincident with or the most approximate to the physical features of the subject 37 to be selected.

That is, in the present embodiment, based on the reference image data of a person in the body position generally coincident with the body position information of the subject inputted through the keyboard 13 and having the physical features coincident with or most approximate to the physical feature information of the subject, among the previously obtained plurality of reference image data, the three-dimensional human body image data is created as described above, and the guide image is displayed on the display apparatus 14.

In the medical guiding system of the present embodiment having such a configuration, a plurality of reference image data by feature previously obtained for different body positions and different physical features are stored in the reference image storage portion 55 as storage means. Then, the operator operates the keyboard 13 as body position information obtaining means and physical feature selection means, and thereby the reference image data, which have been obtained in the body position generally the same as that of the subject 37 and obtained from a person whose physical features coincident with or most approximate to the physical features of the subject 37, are used as the reference image data in creating a guide image.

With such a configuration of the present embodiment, in addition to the effects of the first embodiment, a guide image can be created using the reference image data obtained from a person whose feature points are more approximate to those of the subject 37. Although the size and shape of the organs inside of human bodies are greatly different according to physical features such as the physical size, body height, sex, age, anamnesis, surgical history, and the like, the present embodiment is capable of eliminating incongruity between the reference image data and the anatomical positions and shapes of the viscera and organs of the subject 37 caused by the difference of physical features. Therefore, the medical guiding system according to the present embodiment is capable of making the position of the medical instrument on the guide image coincide with the actual position of the medical instrument with higher accuracy.

Note that, in the present embodiment, the operator selects the reference image data according to the physical features of the subject 37 through the keyboard 13. However, the method of selecting the reference image data according to the physical features of the subject 37 is not limited to the above method. For example, the physical size of the subject 37 may be calculated from the relative positional relationship of the plurality of body surface detection coils 7 mounted on the body surface of the subject 37 on the orthogonal coordinate axis O-xyz so as to automatically select the reference image data approximate most to the physical size of the subject 37.

In addition, information on the physical features of the subject are stored in association with an ID unique to each subject, for example, a medical record number or the like, and by the operator inputting the medical record number through the keyboard 13, the reference image data may be automatically selected based on the physical features of the subject corresponding to the medial record number.

Furthermore, in the present embodiment, there has been shown the example of selecting the reference image data using at least one of the physical size, the body height, the sex, the age, the anamnesis, and the surgical history. However, it is needless to say that other physical features may be used.

(Third Embodiment)

The third embodiment of the present invention is described below. The present embodiment differs in only a part of components and actions from the first embodiment. Therefore, only the different points are described below. In addition, the same components as those in the first embodiment are attached with the same reference numerals and the description thereof will be omitted appropriately.

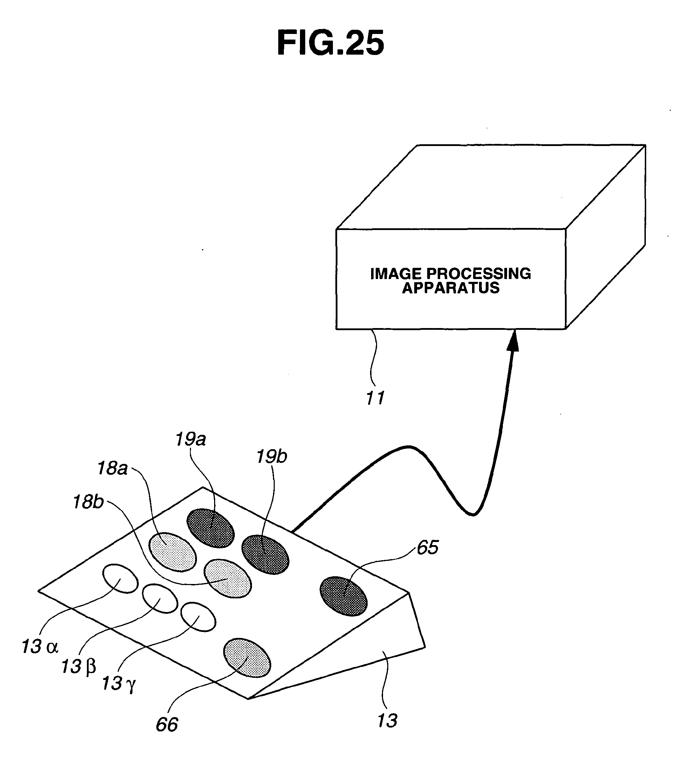

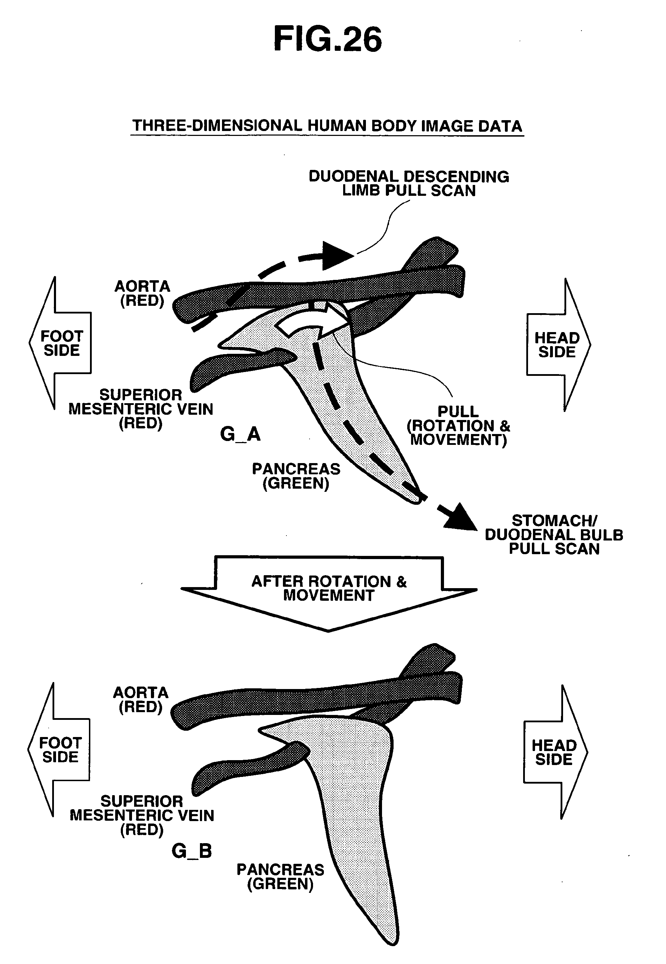

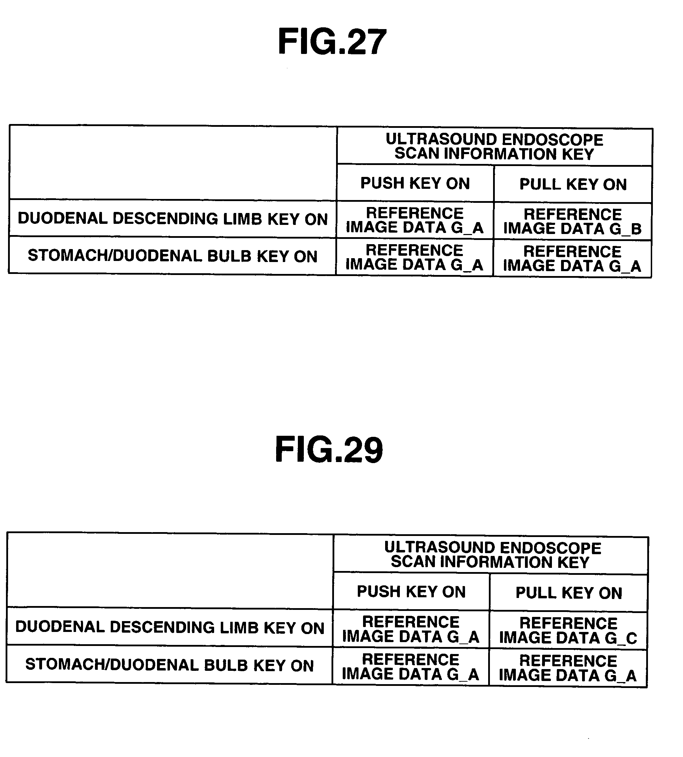

FIG. 25 is an illustration diagram showing a key arrangement of a keyboard. FIG. 26 is an illustration diagram showing rotation and movement of three-dimensional human body image data, and FIG. 27 is an illustration diagram showing correspondence between combination of the keys and reference image data.

As shown in FIG. 25, the component 13 of the present embodiment includes the body cavity feature point specification key 65, the scan control key 66, the display switching keys 13α, 13β, and 13γ, a stomach/duodenal bulb key 18a and a duodenal descending limb key 18b as an ultrasound endoscope scan portion key, and a push key 19a and a pull key 19b as an ultrasound endoscope scan information key.

In the above-described first and second embodiments, the guide image is created by using the reference image data obtained in generally the same body position as that of the subject and the reference image data obtained from the human body whose physical features are generally coincident with or approximate to those of the subject, thereby improving the anatomical degree of coincidence between the guide image and the ultrasound tomographic image displayed on the display apparatus 14.

Here, when diagnosis is performed by introducing the ultrasound endoscope 2, in particular, into the body of the subject, for the following reasons, the positions of the guide image and the ultrasound tomographic image can be anatomically coincided with each other better by using the reference image data created under predetermined conditions.

G_A in FIG. 26 is a three-dimensional human body image data based on the normal reference image data, in which the pancreas and blood vessels are extracted. When inspection is performed in an actual human body using the ultrasound endoscope 2, there are mainly four methods described below.

(First inspection method: duodenal descending limb pull scan)

The first method is one for observing the head of the pancreas (the side close to the aorta in the pancreas in FIG. 26), while pulling the ultrasound endoscope 2 to the mouse side in the duodenal descending limb, which is indicated by the thick dashed line arrow in FIG 26).

(Second inspection method: duodenal descending limb push scan)

The second method is one for observing the head of the pancreas, while pushing the ultrasound endoscope 2 in the duodenal descending limb to the anus side, and the scan direction is opposite to that of the duodenal descending limb pull scan.

(Third inspection method: stomach/duodenal bulb pull scan)

The third method is one for observing the body (near middle part of the pancreas in FIG. 26) and the tail (lower thin side of the pancreas in FIG. 26) of the pancreas, while pulling the ultrasound endoscope 2 to the mouse side from the duodenal bulb toward inside of the stomach, which is indicated by the thick dashed line arrow in FIG. 26). Note that the duodenal bulb and the stomach are located on the nearer side (vertical upper side on the paper surface) than the pancreas.

(Fourth inspection method: stomach/duodenal bulb push scan)

The fourth method is one for observing the body and the tail of the pancreas, while pushing the ultrasound endoscope 2 to the anus side from inside of the stomach toward the duodenal bulb, and the scan direction is opposite to that of the duodenal bulb pull scan. Note that the duodenal bulb and the stomach are located on the nearer side (vertical upper side on the paper surface) than the pancreas.

Among the above methods, the first duodenal descending limb pull scan causes a phenomenon such that the head side of the pancreas is pulled together with the pulling operation of the ultrasound endoscope 2 to the mouse side, to be rotated and moved, as indicated by the block arrow shown in the image data G_A in FIG. 26. When the image of the subject is picked up by the X-ray three-dimensional helical CT apparatus 15 or the three-dimensional MRI apparatus 16, since such a phenomenon is not envisaged, it is difficult to obtain fine anatomical coincidence between the ultrasound tomographic image and the guide image if the reference image data obtained by the normal image pickup method is used. Such a phenomenon does not occur in the second duodenal descending limb push scan, the third stomach/ duodenal bulb pull scan, and the fourth stomach/duodenal bulb push scan.

Therefore, in the reference image storage portion 55 of the present embodiment are previously stored, as the reference data, a plurality of pieces of image data created from a particular subject which correspond to each of a plurality of states of viscera, organs, or tissues. In the present embodiment, the image data of the pancreas in the normal state, the image data of the pancreas in the state where the head of the pancreas is pulled to be rotated and moved are previously stored in the reference image storage portion 55 as part model image data. Methods of creating the data after the rotation and the movement include a plurality of kinds of methods described below.

(First data creation method)

In addition to the normal image pickup by the X-ray three-dimensional helical CT apparatus 15 and the three-dimensional MRI apparatus 16, the ultrasound endoscope 2 is inserted and an image is picked up again by the X-ray three-dimensional helical CT apparatus 15 and the three-dimensional MRI apparatus 16, while pulling the head of the pancreas by the duodenal descending limb pull scan. Thus, the reference image data of the pancreas in the normal state and the reference image data after the pancreas has been rotated and moved can be obtained.

(Second data creation method)

After the normal image pickup with respect to a particular subject by the X-ray three-dimensional helical CT apparatus 15 and the three-dimensional MRI apparatus 16, image pickup is performed with respect to the subject by the ultrasound endoscope 2, to create new reference image data by rotating and moving the pancreas in the reference image data such that the guide image and the ultrasound tomographic image coincide with each other. Thus, the reference image data of the pancreas in the normal state and the reference image data after the pancreas has been rotated and moved can be obtained.

In the second data creation method, the three-dimensional human body image data G_B is created in the following procedure.

First, the operator inputs the moving direction, the moving distance, and the rotation angle of the pancreas via the keyboard 13 or the mouse 12. The three-dimensional human body image creation circuit 57 creates, based on the inputted moving direction, moving distance, and rotation angle, the three-dimensional human body image data from the original reference image data by rotating and moving the pancreas.

Next, the synthesis circuit 58 synthesizes the three-dimensional human body image data of the rotated and moved pancreas, the image index data, and the insertion shape data, to create synthetic three-dimensional data. The synthetic three-dimensional data is displayed on the display apparatus 14, through the rotational transformation circuit 59, the three-dimensional guide image creation circuit A, the three-dimensional guide image creation circuit B, and the mixing circuit 61.

The operator compares anatomical degree of coincidence between the ultrasound tomographic image and the two three-dimensional guide images on the display screen of the display apparatus 14. Then, the operator again inputs the moving direction, the moving distance, and the rotation angle of the pancreas through the keyboard 13 and the mouse 12 such that the ultrasound tomographic image and the two three-dimensional guide images anatomically coincide well with each other. Then the above-described procedure is repeated.

Since the working of the present embodiment has no change even if either reference image data created by the first or second method is used, the working of the present embodiment will be described premised on the second method.

In the present embodiment, the reference image data of the pancreas in the normal state and the reference image data after the pancreas has been rotated and moved are stored in the reference image storage portion 55. The three-dimensional human body image data based on the reference image data of the pancreas in the normal state is shown in G_A in FIG 26, and the three-dimensional human body image data based on the reference image data after the pancreas has been rotated and moved is shown in G_B in FIG. 26.

The selection between the reference image data of the pancreas in the normal state and the reference image data after the pancreas has been rotated and moved is performed through the keyboard 13 and the mouse 12, and the control circuit 63, as the state selection portions. In the present embodiment, the operator presses either one of the push key 19a and the pull key 19b as the ultrasound endoscope scan information keys on the keyboard 13. When one of these keys 19a, 19b is turned on, the other is turned off. In addition, the operator presses either one of the stomach/duodenal bulb key 18a or the duodenal descending limb key 18b as the endoscope scan portion keys on the keyboard 13. When one of these keys 18a, 18b is turned on, the other is turned off.

Determination whether to use either the three-dimensional human body image data G_A or the three-dimensional human body image data G_B in FIG. 26 is made by the control circuit 63 based on the on and off combination of the keys 18a, 18b, and the keys 19a, 19b. The determination by the control circuit 63 is based on the table in FIG. 27. That is, when the duodenal descending limb key 18b and the push key 19a are turned on, when the stomach/duodenal bulb key 18a and the push key 19a are turned on, and when the stomach/duodenal bulb key 18a and the pull key 19b are turned on, the control circuit 63 selects the image data G_A as the reference image data. On the other hand, when the duodenal descending limb key 18b and the pull key 19b are turned on, the control circuit 63 selects the image data G_B as the reference image data.

In response to the instruction from the control circuit 63, the interpolation circuit 56 reads the reference image data again. Thus, the voxel spaces in the interpolation memory and the synthesis memory are filled with the reference image data read corresponding to the combination of the keys indicated in the table in FIG. 27, and the three-dimensional human body image data, the synthetic three-dimensional data, and the three-dimensional guide image data are replaced.

All of the control circuit 63, the interpolation circuit 56, the three-dimensional human body image creation circuit 57, the synthesis circuit 58, the rotational transformation circuit 59, the three-dimensional guide image creation circuits A, B, and the mixing circuit 61 work in real time, so that the guide image is instantaneously changed over in response to the key operation by the operator.

Thus, the operator can obtain anatomically excellent coincidence between the ultrasound tomographic image and the guide image, whether it be the duodenal descending limb or the stomach/duodenal bulb, or whether it be the push scan or the pull scan of the ultrasound endoscope 2.

With the above-described present embodiment, the reference image storage portion 55 stores the reference image data of the pancreas in the normal state and the reference image data after the pancreas has been rotated or moved, the control circuit 63 determines whether to use, as the reference image data, either the three-dimensional image data G_A or the three-dimensional image data G_B in FIG. 26, the interpolation circuit 56 reads the reference image data again in response to the instruction from the control circuit 63, and the voxel spaces in the interpolation memory and the synthesis memory are filled with the reference image data read corresponding to the combination of keys indicated in the table in FIG. 27, such that the three-dimensional human body image data and the guide image are instantaneously changed over in response to the key operation by the operator. Accordingly, the guide image accurately showing the actual anatomical position and orientation of the ultrasound tomographic image can be displayed. Since the head of the pancreas is rotated and moved in the duodenal descending limb pull scan, in particular, the effect is noteworthy.

In addition, in the present embodiment, the image index creation circuit 52 creates the image index data by synthesizing the ultrasound tomographic image marker Mu with the blue distal end direction marker Md and the arrow-shaped yellowish green six o'clock direction marker Mt, the synthesis circuit 58 synthesizes the three-dimensional human body image data, the image index data, and the insertion shape data in the same voxel space, the mixing circuit 61 creates mixing data for adjacently displaying the ultrasound tomographic image data from the ultrasound observation apparatus 4 and the three-dimensional guide image data, the display circuit 62 converts the mixing data into an analog video signal, and the display apparatus 14 adjacently displays the ultrasound tomographic image and the three-dimensional guide image based on the analog video signal.

Therefore, the present embodiment can guide the positional relationship between the ultrasound tomographic image and the region of interest such as the pancreas and also how the radial scanning surface of the ultrasound endoscope, the flexible portion, and the rigid portion are oriented and shaped with respect to the body cavity wall such as the gastrointestinal tract and the like. Therefore, the operator can visually grasp these relationships, thereby facilitating the diagnosis, treatment, and the like with respect to the region of interest.

Furthermore, in the present embodiment, the matching circuit 51 repeats the processings described in the steps S4-4 to S4-9 to load the position/orientation data at the moment that the mixing circuit 61 has loaded the ultrasound tomographic image data, and combines the first conversion equation and the translation by the vector $P'P''$ to newly create a second conversion equation expressing the second mapping, and converts the directional components $(x0, y0, z0)$ of the position vector $OO''$ of the center of the ultrasound tomographic image $O''$ on the orthogonal coordinate axis $O\text{-}xyz$, the angular components $(\psi, \theta, \phi)$ of the Euler angle indicating the orientation of the image position/orientation detection coil 31 with respect to the orthogonal coordinate axis $O\text{-}xyz$, and the directional components $(xi, yi, zi)$ of the position vectors of each of the plurality of insertion shape detection coils 32, on the orthogonal coordinate axis $O\text{-}xyz$ (i is a natural number between 1 and the total number of the insertion shape detection coils 32), into position/orientation mapping data.

Therefore, the present embodiment has such an effect that the ultrasound tomographic image, the flexible portion 22, the rigid portion 21 are coincided with the ultrasound tomographic image marker Mu, the distal end direction marker Md, the six o'clock direction marker Mt, and the insertion shape marker Ms on the three-dimensional guide image with higher anatomical accuracy, even if the body position of the subject 37 is changed during the inspection by the ultrasound endoscope 2.

Furthermore, images are normally picked up with a patient being in a supine position when using the X-ray three-dimensional helical CT apparatus 15 and the three-dimensional MRI apparatus 16, so that the body position of the patient is different from the left lateral position in the ultrasound endoscopy. However, in the present embodiment, the matching circuit 51 combines the first mapping with the translation by the vector $P'P''$ as a correction value to create a second conversion equation expressing the second mapping. Therefore, with the present embodiment, even if the various organs in the subject 37 move with respect to the reference image data according to the change of the posture at the time of ultrasound endoscopy, the anatomical position of the ultrasound tomographic image in the subject 37 and the anatomical position of the ultrasound tomographic image on the three-dimensional guide image can be accurately coincided with each other. Therefore, the three-dimensional guide image can guide the ultrasound tomographic image more accurately.

In addition, according to the present embodiment, the three-dimensional guide image creation circuit A creates three-dimensional guide image data of when the subject is observed from the ventral side direction with the head side of the subject setting on the right side and the foot side of the subject setting on the left side. In the ultrasound endoscopy, the subject 37 is normally inspected in the left lateral body position, and the three-dimensional guide image is also displayed in the left lateral position, so that it is easy to compare the subject and the three-dimensional guide image. This allows the operator to easily understand the three-dimensional guide image, thereby improving and properly supporting the operability during diagnosis, treatment, or the like by the operator.

Furthermore, with the present embodiment, the three-dimensional guide image creation circuits A , B create three-dimensional guide images with the line of sight set in different directions, so that it is possible to guide the positional relationship between the ultrasound tomographic image and the region of interest such as the pancreas from a plurality of directions, and guide also how the ultrasound tomographic image and the flexible portion 22 and the rigid portion 21 of the ultrasound endoscope 2 are oriented and shaped with respect to the body cavity wall of gastrointestinal tract and the like from a plurality of directions. This allows the operator to easily understand the images.

In addition, according to the present embodiment, the three-dimensional guide image creation circuit B creates, based on the position/orientation mapping data, the three-dimensional guide images on which the normal line of the ultrasound tomographic image marker Mu is set so as to coincide with the observation line of sight, that is, the normal line of the screen of the display apparatus 14 and the 6 o'clock direction marker Mt is set so as to orient downward on the screen of the display apparatus 14. This allows the direction of the three-dimensional image to coincide with that of the ultrasound tomographic image displayed in real time next to the three-dimensional guide image on the screen of the display apparatus 14. Therefore, the operator can easily compare these images with each other and anatomically interpret the ultrasound tomographic image.

In particular, the three-dimensional guide image of when the subject is observed from the ventral side is virtually a wide-range guide image and the three-dimensional guide image of when the subject is observed from the same direction as that of the ultrasound tomographic image is a detailed guide image, so that the operator can grasp anatomically rough position in the former and fine-tunes the scanning surface of the ultrasound while obtaining detailed anatomical interpretation of the ultrasound tomographic image in the latter. This enables inspection to be efficient.

In addition, according to the present embodiment, the three-dimensional guide image creation circuit B creates a three-dimensional guide image data such that, out of the two areas separated by the ultrasound tomographic image marker Mu among the image index data, an area of the distal end side of the flexible portion 22, that is, the area on the screen of the display apparatus 14 which is closer to the operator is not displayed and the luminance of the area on the ultrasound tomographic image marker Mu is different from that of the area behind the ultrasound tomographic image marker Mu. Therefore, the viscera displayed on the side closer to the operator does not disturb the observation by the operator, thereby allowing the operator to more easily compare the three-dimensional guide image with the ultrasound tomographic image displayed in real time next to the three-dimensional guide image on the screen of the display apparatus 14. Therefore, it is easy for the operator to anatomically interpret the ultrasound tomographic image.

(Fourth Embodiment)

The fourth embodiment of the present invention will be described below. The present embodiment differs in only a part of components and actions from the third embodiment. Therefore, only the different points are described below. In addition, the same components as those in the first embodiment are attached with the same reference numerals and the description thereof will be omitted appropriately.

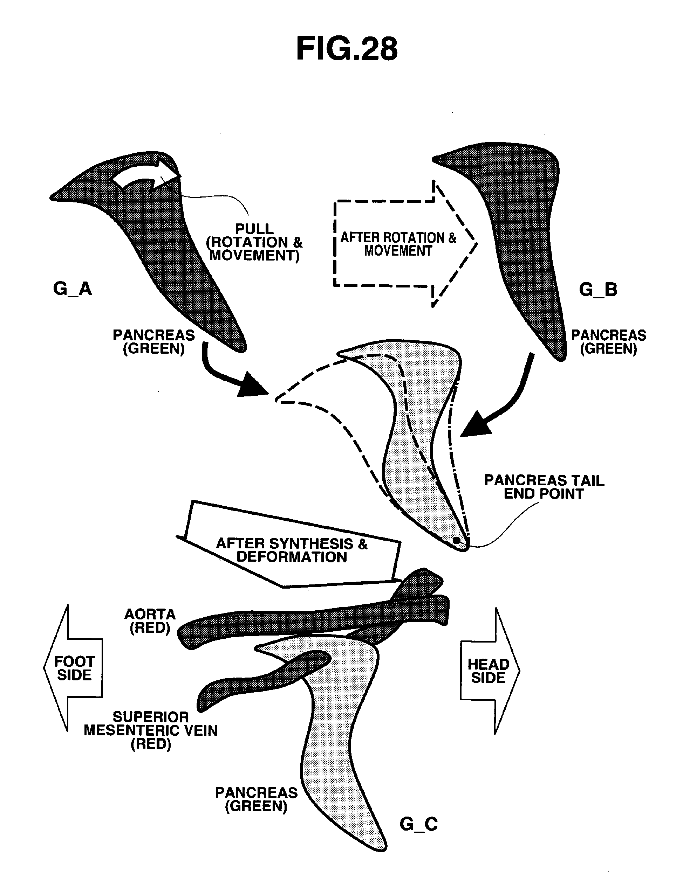

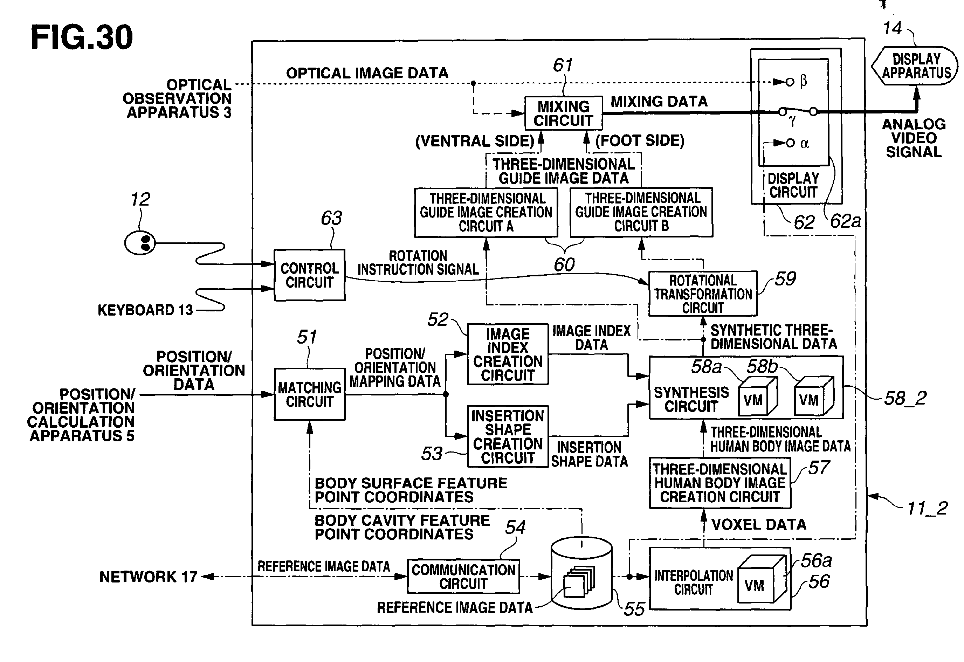

FIG. 28 is an illustration diagram showing synthesis/deformation of the three-dimensional human body image data, FIG. 29 is an illustration diagram showing correspondence between combination of keys and the three-dimensional human body image data, and FIG. 30 is a block diagram showing a configuration of an image processing apparatus.

The fourth embodiment differs from the third embodiment in the configuration of the synthesis circuit 58 of the image processing apparatus 11. As shown in FIG. 30, a synthesis circuit 58_2 of an image processing apparatus 11_2 of the present embodiment includes one more additional synthesis memory (volume memory) 58b, compared with the synthesis memory 58 in the first embodiment.

The additional volume memory makes the working of the fourth embodiment differ from that of the third embodiment in the workings of the reference image storage portion 55 and the three-dimensional human body image creation circuit 57.

In the third embodiment, the reference image storage portion 55 stores the reference image data of the pancreas in the normal state and the reference image data after the pancreas has been rotated and moved, and the control circuit 63 determines whether to use either the three-dimensional human body image data G_A or the three-dimensional human body image data G_B in FIG. 26.

On the other hand, in the fourth embodiment, the three-dimensional human body image creation circuit 57 causes one of the synthesis memories 58a, 58b of the synthesis circuit 58_2 to store the three-dimensional human body image data G_A based on the reference image data of the pancreas in the normal state shown in FIG. 26.

Furthermore, the three-dimensional human body image creation circuit 57 creates, as shown in FIG. 28, three-dimensional human body image data G_C by synthesizing and deforming the three-dimensional human body image data G_A and the three-dimensional human body image data G_B based on the reference image data after the pancreas has been rotated and moved. The three-dimensional human body image data G_C is newly created from the three-dimensional human body image data G_A and G_B, and includes deformed hypothetical pancreas.

In FIG. 28, for the convenience of the description, the positions of the pancreas are adjusted at the pancreas tail end point as one example. The tail end point is used in the description, because the tail end portion is the most stable point with minimum rotation and movement in the pancreas even in a case of the duodenal descending limb pull scan.

The method of creating the three-dimensional human body image data G_C by synthesizing and deforming the above-mentioned three-dimensional human body image data G_A and G_B is as follows.

The operator inputs the moving direction, moving distance, and the rotation angle of the pancreas through the keyboard 13 or the mouse 12. The three-dimensional human body image creation circuit 57 creates the three-dimensional human body image data from the original reference image data by rotating and moving the pancreas based on the inputted moving direction, the moving distance, and the rotational angle.

Next, the synthesis circuit 58_2 creates synthetic three-dimensional data by synthesizing the three-dimensional human body image data after the pancreas has been rotated and moved, the image index data, and the insertion shape data. The synthetic three-dimensional data is displayed on the display apparatus 14 via the rotational transformation circuit 59, the three-dimensional guide image creation circuits A, B, and the mixing circuit 61.

The operator compares the degree of anatomical coincidence between the ultrasound tomographic image and the two three-dimensional guide images on the display screen of the display apparatus 14. Then, the operator again inputs the moving direction, the moving distance, and the rotation angle of the pancreas through the keyboard 13 or the mouse 12 such that the ultrasound tomographic image and the two three-dimensional guide images anatomically coincide well each other.

Then, the operator repeats the above-described operation to create a three-dimensional guide image. The procedure thus far described is the same as that in the third embodiment.

Moreover, the operator allows the three-dimensional guide images to be displayed on the display apparatus 14 so as to be superimposed one another through the keyboard 13 or the mouse 12, as shown in FIG. 28. Then, the operator makes a tracing on the display screen through the keyboard 13 or the mouse 12 such that the pancreas becomes an appropriate shape.

The three-dimensional human body image creation circuit 57 creates three-dimensional human body image data G_C again based on the traced information to cause one of the synthesis memories 58a, 58b of the synthesis circuit 58_2 to store the created three-dimensional human body image data G_C.

Determination whether to use either the three-dimensional human body image data G_A in FIG. 26 or the three-dimensional human body image data G_C in FIG. 28 is made by the control circuit 63 according to the state of on and off combination of the ultrasound endoscope scan portion keys and the ultrasound endoscope scan information keys.

The determination by the control circuit 63 is based on the table in FIG. 29. That is, when the duodenal descending limb key 18b and the push key 19a are turned on, when the stomach/duodenal bulb key 18a and the push key 19a are turned on, and when the stomach/duodenal bulb key 18a and the pull key 19b are turned on, the control circuit 63 selects the image data G_A as the reference image data. On the other hand, when the duodenal descending limb key 18b and the pull key 19b are turned on, the control circuit 63 selects the image data G_C created by synthesizing and deforming the three-dimensional human body image data G_A and the three-dimensional human body image data G_B, as the reference image data.

In response to the instruction from the control circuit 63, the synthesis circuit 58_2 changes over between the three-dimensional human body image data G_A and the three-dimensional human body image data G_C as the three-dimensional human body image data to be synthesized with the image index data and the insertion shape data.

The three-dimensional human body image data, the synthetic three-dimensional data, and the three-dimensional guide image data are thus replaced. Each of the control circuit 63, the three-dimensional human body image creation circuit 57, the synthesis circuit 58_2, the rotational transformation circuit 59, the three-dimensional guide image creation circuit A, the three-dimensional guide iamge creation circuit B, and the mixing circuit 61 works in real time, so that the guide image is instantaneously changed over in response to the key operation by the operator.

Thus, the operator can obtain anatomically excellent coincidence between the ultrasound tomographic image and the guide image, whether it be the duodenal descending limb or the stomach/duodenal bulb, or whether it be the push scan of the ultrasound endoscope 2 or the pull scan of the ultrasound endoscope 2. Other workings are the same as those in the third embodiment.

In the fourth embodiment, the three-dimensional human body image creation circuit 57 causes one of the two synthesis memories 58a, 58b of the synthesis circuit 58_2 to store the three-dimensional human body image data G_A based on the reference image data of the pancreas in the normal state shown in FIG. 26, to create the three-dimensional human body image data G_C by synthesizing and deforming the three-dimensional human body image data G_A and the three-dimensional human body image data G_B based on the reference image data after the pancreas has been rotated and moved, and causes the other of the two memories 58a, 58b of the synthesis circuit 58_2 to store the three-dimensional human body image data G_C.

Then, the control circuit 63 determines whether to use either the three-dimensional human body image data G_A in FIG. 26 or the three-dimensional human body image data G_C in FIG. 28 from the state of on and off combination of the ultrasound endoscope scan portion key and the ultrasound endoscope scan information key. In response to the instruction from the control circuit 63, the synthesis circuit 58_2 changes over between the three-dimensional human body image data G_A and the three-dimensional human body image data G_C to select as the three-dimensional human image data to be synthesized with the image index data and the insertion shape data, and the synthetic three-dimensional data and the three-dimensional guide image data are replaced. In response to the key operation by the operator, the guide image is instantaneously changed.

Therefore, unlike the third embodiment, it is unnecessary to read the reference image data to create three-dimensional human body image data again in the fourth embodiment, so that a guide image correctly showing the actual anatomical position and orientation of the ultrasound tomographic image can be obtained and displayed at higher speed.

In addition, because the deformation of the pancreas due to the duodenal descending limb pull scan mainly occurs at the head portion of the pancreas and does not occur at the tail portion, the guide image closer to the deformed actual pancreas than in the third embodiment can be displayed. Other effects are the same as those in the third embodiment.

Having described the preferred embodiments of the invention referring to the accompanying drawings, it should be understood that the present invention is not limited to those precise embodiments and various changes and modifications thereof could be made by one skilled in the art without departing from the spirit or scope of the invention as defined in the appended claims.

**Claims**

1. A medical guiding system comprising:

    a detection portion for detecting at least one of a position and an orientation of a medical instrument;
    a storage portion for storing a plurality of reference image data, the plurality of reference image data being obtained from a human body in a plurality of states before the medical instrument is used with respect to a subject, and including anatomical positional information of at least one of a viscus and an organ that corresponds to each of the plurality of states;
    a state selection portion for selecting, among the plurality of reference image data stored in the storage portion, the reference image data obtained in a state coincident with or approximate to a state of the subject in using the medical instrument; and
    a guide image creation portion for creating a guide image showing at least one of an anatomical position, shape, and orientation of the medical instrument with respect to the subject, based on at least one of the position and the orientation of the medical instrument detected by the detection portion and the reference image data stored in the state selection portion.

2. The medical guiding system according to claim 1, wherein
    the plurality of reference image data stored in the storage portion are obtained in states where a human body is set in a plurality of body positions different from one another, and
    the state selection portion selects the reference image data obtained from a human body in a body position coincident with or approximate to a body position of the subject in using the medical instrument.

3. The medical guiding system according to claim 2, wherein
    the medical instrument is an ultrasound probe to be introduced into a body of the subject, for scanning ultrasound to obtain an ultrasound signal for creating an ultrasound tomographic image, and
    the reference image data is obtained at least in a state where the human body is set in a left lateral position.

4. The medical guiding system according to claim 1, wherein
    the plurality of reference image data stored in the storage portion are obtained, concerning a predetermined one or a plurality of physical features, from a plurality of human bodies corresponding to a plurality of different conditions of the one or the plurality of physical features, and
    the state selection portion selects the reference image data obtained from the human bodies having the physical feature coincident with or approximate to a physical feature of the subject in using the medical instrument.

5. The medical guiding system according to claim 4, wherein the physical feature includes at least one of body size, body height, sex, age, anamnesis, and surgical history.

6. The medical guiding system according to claim 4, wherein the medical instrument is an ultrasound probe to be introduced into a body of the subject, for scanning ultrasound to obtain an ultrasound signal for creating an ultrasound tomographic image.

7. The medical guiding system according to claim 1, wherein
    the plurality of reference image data stored in the storage portion are obtained, concerning at least one of a viscus and an organ, from a human body in a plurality of states where at least one of a position, a shape, and an orientation is different, and
    the state selection portion selects the reference image data obtained in a state coincident with or approximate to at least one of the position, the shape, and the orientation of at least one of the viscus and the organ of the subject.

**8.** The medical guiding system according to claim 7, wherein
the medical instrument is an ultrasound probe to be introduced into a body of the subject, for scanning ultrasound to obtain an ultrasound signal for creating an ultrasound tomographic image, and
the state selection portion selects the reference image data based on at least one of a scanning position, a moving direction and a moving trajectory of the ultrasound probe.

**9.** The medical guiding system according to claim 8, wherein the scanning position is at least one of an esophagus, a stomach, a duodenum, a duodenal bulb, a duodenal descending limb, and a duodenal transverse limb.

**10.** The medical guiding system according to claim 9, wherein the moving direction is a direction pulling out or pushing in the ultrasound probe in a body of the subject.

**11.** The medical guiding system according to claim 7, further including a human body image creation portion for changing at least one of a position, a shape, and an orientation of a predetermined viscus or an organ in the reference image data selected by the state selection portion to create a human body image, wherein
the guide image creation portion creates a guide image showing at least one of an anatomical position, shape, and orientation of the medical instrument with respect to the subject, based on at least one of the position and orientation of the medical instrument detected by the detection portion and the human body image created by the human body image creation portion.

**12.** The medical guiding system according to claim 11, wherein
the medical instrument is an ultrasound probe to be introduced into a body of the subject, for scanning ultrasound to obtain an ultrasound signal for creating an ultrasound tomographic image, and
the human body image creation portion changes at least one of the position, the shape, and the orientation of the predetermined viscus or the organ to create the human body image, based on at least one of a scanning position, a moving direction and a moving trajectory of the ultrasound probe.

**13.** The medical guiding system according to claim 12, wherein the scanning position is at least one of an esophagus, a stomach, a duodenum, a duodenal bulb, a duodenal descending limb, and a duodenal transverse limb.

**14.** The medical guiding system according to claim 13, wherein the moving direction is a direction pulling out or pushing in the ultrasound probe in a body of the subject.

**15.** The medical guiding system according to claim 11, further including a synthesis portion for synthesizing the reference image data selected by the state selection portion with the human body image in which at least one of the position, the shape, and the orientation of the predetermined viscus or the organ in the reference image data is changed, to create a new human body image.

**16.** The medical guiding system according to claim 15, wherein
the medical instrument is an ultrasound probe to be introduced into a body of the subject, for scanning ultrasound to obtain an ultrasound signal for creating an ultrasound tomographic image, and
the human body image creation portion changes at least one of the position, the shape, and the orientation of the predetermined viscus or the organ to create the human body image, based on at least one of a scanning position, a moving direction and a moving trajectory of the ultrasound probe.

**17.** The medical guiding system according to claim 16, wherein the scanning position is at least one of an esophagus, a stomach, a duodenum, a duodenal bulb, a duodenal descending limb, and a duodenal transverse limb.

**18.** The medical guiding system according to claim 17, wherein the moving direction is a direction pulling out or pushing in the ultrasound probe in a body of the subject.

**FIG.1**

ULTRASOUND BEAM

$V_{12}$

$V$

$V_3$

CCD SIGNAL

OPTICAL OBSERVATION APPARATUS *3*

ECHO SIGNAL

ULTRASOUND OBSERVATION APPARATUS *4*

DISPLAY APPARATUS *1* *14*

ULTRASOUND TOMOGRAPHIC IMAGE DATA

OPTICAL IMAGE DATA

ANALOG VIDEO SIGNAL

SCAN CONTROL SIGNAL

ALTERNATING MAGNETIC FIELD

TRANSMISSION ANTENNA *6*

POSITION ELECTRIC SIGNAL

POSITION/ ORIENTATION CALCULATION APPARATUS *5*

A/D *9c*

A/D *9b*

A/D *9a*

*9*

*36*

*35*

*34*

POSITION/ ORIENTATION DATA

*33*

IMAGE PROCESSING APPARATUS *11*

*12*

*13*

*17*

REFERENCE IMAGE DATA

THREE-DIMENSIONAL MRI APPARATUS *16*

X-RAY THREE-DIMENSIONAL HELICAL CT APPARATUS *15*

EP 2 036 494 A2

# FIG.2

RIGHT ANTERIOR
SUPERIOR ILIAC SPINE

*37*

7

7

7

XIPHOID
PROCESS

SPINOUS PROCESS OF VERTEBRAL BODY

LEFT ANTERIOR SUPERIOR ILIAC SPINE

PILLOW

BED

*38*

# FIG.3

*42*  *41*  *8*

SIGNAL LINE

TO POSITION/ORIENTATION
CALCULATION APPARATUS 5

*43*

**FIG.4**

# FIG.5

# FIG.6

# FIG.7

IMAGE PROCESSING
APPARATUS

*11*

*67*

*65*

*13α*

*13β*

*13γ*

*66*

*13*

# FIG.8

EP 2 036 494 A2

# FIG.9

XIPHOID PROCESS
(ON n1-th)

RIGHT ANTERIOR
SUPERIOR ILIAC SPINE
(ON n2-th)

VECTOR
Q'Q"=P'P"

O y → x

z

ULTRASOUND
ENDOSCOPE

REFERENCE IMAGE DATA

Q'    Q"

P' → P"

n1-th

n2-th

37

7

XIPHOID
PROCESS

y'

O'    x'

z'

7

7

7    7

O"

P

SPINOUS
PROCESS OF
VERTEBRAL
BODY

SPINOUS
PROCESS OF
VERTEBRAL
BODY
(ON n2-th)

LEFT ANTERIOR
SUPERIOR
ILIAC SPINE
(ON n2-th)

LEFT ANTERIOR
SUPERIOR
ILIAC SPINE

BODY CAVITY
CONTACT PROBE

MAPPING
(P→P')

DUODENAL
PAPILLA

RIGHT ANTERIOR
SUPERIOR
ILIAC SPINE

# FIG.10

MARKER CREATION

IMAGE INDEX DATA

# FIG.11

POSITION OF INSERTION
SHAPE DETECTION COIL

POSITION OF
POSITION/ORIENTATION
DETECTION COIL

(x4, y4, z4)

(x3, y3, z3)

(x2, y2, z2)

(x1, y1, z1)

(x0, y0, z0)

z

y

O

x

INTERPOLATION
MARKER CREATION

INSERTION SHAPE DATA

Ms

Mc

z'

y'

O'

x'

# FIG.12

THREE-DIMENSIONAL HUMAN BODY IMAGE DATA

FOOT SIDE

AORTA (RED)

HEAD SIDE

SUPERIOR MESENTERIC VEIN (RED)

PANCREAS (GREEN)

# FIG.13

IMAGE INDEX DATA

Mc

z'

Mu

Md

Mt

Ms

y'

O'

INSERTION SHAPE DATA

x'

# FIG.14

**THREE-DIMENSIONAL GUIDE IMAGE DATA**

**(OBSERVE FROM VENTRAL SIDE OF SUBJECT)**

**(ULTRASOUND TOMOGRAPHIC IMAGE MARKER: OPAQUE DISPLAY)**

# FIG.15

THREE-DIMENSIONAL GUIDE IMAGE DATA

(SAME DIRECTION AS THAT OF ULTRASOUND TOMOGRAPHIC IMAGE, OBSERVE FROM -V DIRECTION)

(ULTRASOUND TOMOGRAPHIC IMAGE MARKER: TRANSLUCENT DISPLAY)

# FIG.16

WIDE RANGE GUIDE IMAGE
(FROM VENTRAL SIDE)

Mu

Mu

ULTRASOUND
TOMOGRAPHIC IMAGE

DETAILED GUIDE IMAGE
(SAME DIRECTION AS
THAT OF ULTRASOUND
TOMOGRAPHIC IMAGE)

DUODENAL
WALL

AORTA

SUPERIOR
MESENTERIC
VEIN

PANCREAS

# FIG.17

START

BODY SURFACE FEATURE POINT AND BODY
CAVITY FEATURE POINT SPECIFICATION
PROCESSING ON REFERENCE IMAGE DATA — S-1

FIX BODY SURFACE DETECTION COIL
TO SUBJECT — S-2

CORRECTION VALUE CALCULATION
PROCESSING — S-3

ULTRASOUND TOMOGRAPHIC IMAGE AND
THREE-DIMENSIONAL GUIDE IMAGE CREATION/
DISPLAY PROCESSING — S-4

END

# FIG.18

BODY SURFACE FEATURE POINT AND
BODY CAVITY FEATURE POINT SPECIFICATION
PROCESSING ON REFERENCE IMAGE DATA

S-1-1 — PRESS DISPLAY SWITCHING KEY α ← DISPLAY SWITCHING KEY 13 α

S-1-2 — SPECIFY REFERENCE IMAGE DATA ← MOUSE 12 KEYBOARD 13

S-1-3 — DISPLAY REFERENCE IMAGE DATA

S-1-4 — SPECIFY BODY SURFACE FEATURE POINTS ON REFERENCE IMAGE DATA ← MOUSE 12 KEYBOARD 13

S-1-5 — SPECIFY BODY CAVITY FEATURE POINT P" ON REFERENCE IMAGE DATA ← MOUSE 12 KEYBOARD 13

S-1-6 — STORE COORDINATES IN VOXEL SPACE OF EACH FEATURE POINT

RETURN

# FIG.19

CORRECTION VALUE
CALCULATION PROCESSING

S-3-1
PRESS DISPLAY SWITCHING KEY β ◄─── DISPLAY SWITCHING KEY 13 β

S-3-2
DISPLAY OPTICAL IMAGE

S-3-3
INSERT RIGID PORTION AND
FLEXIBLE PORTION INTO BODY CAVITY

S-3-4
MOVE RIGID PORTION TO SEARCH
FOR BODY CAVITY FEATURE POINT

S-3-5
PROJECT BODY CAVITY CONTACT
PROBE FROM PROJECTION PORT
TO BRING BODY CAVITY CONTACT
PROBE INTO CONTACT WITH BODY
CAVITY FEATURE POINT

S-3-6
PRESS BODY CAVITY FEATURE ◄─── FEATURE POINT
POINT SPECIFICATION KEY        SPECIFICATION
                               KEY 65

S-3-7
LOAD POSITION/          POSITION/
ORIENTATION DATA   ◄─── ORIENTATION
                        CALCULATION
                        APPARATUS 5

S-3-8
CREATE FIRST CONVERSION
EQUATION EXPRESSING FIRST
MAPPING FROM COORDINATES OF
BODY SURFACE FEATURE POINTS

S-3-9
MAP BODY CAVITY FEATURE
POINT P TO POINT P' IN VOXEL
SPACE BASED ON FIRST
CONVERSION EQUATION

S-3-10
CALCULATE VECTOR P' P"

S-3-11
STORE VECTOR P' P" AS
CORRECTION VALUE

RETURN

45

# FIG.20

DISPLAY SCREEN (OPTICAL IMAGE)

DUODENAL PAPILLA P

BODY CAVITY CONTACT PROBE 8

# FIG.21

( ULTRASOUND TOMOGRAPHIC IMAGE
AND THREE-DIMENSIONAL GUIDE IMAGE
CREATION/DISPLAY PROCESSING )

S4-1 — PRESS DISPLAY SWITCHING KEY ◄— DISPLAY SWITCHING KEY γ

S4-2 — PRESS SCAN CONTROL KEY ◄— SCAN CONTROL KEY 66

S4-3 — START RADIAL SCAN

S4-4 — LOAD ULTRASOUND
TOMOGRAPHIC IMAGE DATA ◄— ULTRASOUND
OBSERVATION
APPARATUS 4

S4-5 — LOAD POSITION/
ORIENTATION DATA ◄— POSITION/
ORIENTATION
CALCULATION
APPARATUS 5

S4-6 — COMBINE FIRST CONVERSION
EQUATION WITH TRANSLATION
BY VECTOR P' P" TO CREATE
NEW SECOND CONVERSION
EQUATION EXPRESSING
SECOND MAPPING

S4-8 — CREATE THREE-DIMENSIONAL
IMAGE DATA

S4-7 — CONVERT POSITION/
ORIENTATION DATA INTO
POSITION/ORIENTATION
MAPPING DATA

S4-9 — DISPLAY ULTRASOUND
TOMOGRPHIC IMAGE AND
THREE-DIMENSIONAL
GUIDE IMAGE

S4-10 — RADIAL SCAN END? — NO

YES

( RETURN )

# FIG.22

O'
y'
z'
x'

PORTAL VEIN
GALLBLADDER
INFERIOR
VENA CAVA
AORTA
DORSAL SIDE
VENTRAL SIDE
PANCREAS
G

# FIG.23

INFERIOR
VENA CAVA
PORTAL
VEIN
GALLBLADDER
AORTA
DORSAL
SIDE
VENTRAL
SIDE
PANCREAS
y'
O' z'
x'
G

# FIG.24

IMAGE PROCESSING
APPARATUS

11

67

68

65

13 α

13 β

13 γ

66

13

# FIG.25

IMAGE PROCESSING
APPARATUS

11

19a

18a

19b

18b

65

13 α

13 β

13 γ

66

13

# FIG.26

THREE-DIMENSIONAL HUMAN BODY IMAGE DATA

DUODENAL DESCENDING
LIMB PULL SCAN

AORTA
(RED)

FOOT
SIDE

HEAD
SIDE

SUPERIOR
MESENTERIC VEIN
(RED)

PULL
(ROTATION &
MOVEMENT)

G_A

PANCREAS
(GREEN)

STOMACH/
DUODENAL BULB
PULL SCAN

AFTER ROTATION &
MOVEMENT

AORTA
(RED)

FOOT
SIDE

HEAD
SIDE

SUPERIOR
MESENTERIC VEIN
(RED)

G_B

PANCREAS
(GREEN)

# FIG.27

|  | ULTRASOUND ENDOSCOPE SCAN INFORMATION KEY | |
| --- | --- | --- |
|  | PUSH KEY ON | PULL KEY ON |
| DUODENAL DESCENDING LIMB KEY ON | REFERENCE IMAGE DATA G_A | REFERENCE IMAGE DATA G_B |
| STOMACH/DUODENAL BULB KEY ON | REFERENCE IMAGE DATA G_A | REFERENCE IMAGE DATA G_A |

# FIG.29

|  | ULTRASOUND ENDOSCOPE SCAN INFORMATION KEY | |
| --- | --- | --- |
|  | PUSH KEY ON | PULL KEY ON |
| DUODENAL DESCENDING LIMB KEY ON | REFERENCE IMAGE DATA G_A | REFERENCE IMAGE DATA G_C |
| STOMACH/DUODENAL BULB KEY ON | REFERENCE IMAGE DATA G_A | REFERENCE IMAGE DATA G_A |

# FIG.28

## FIG.30

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005312770 A
- JP 2006149481 A
- WO 2006057296 A
- JP 2007037790 A
- JP 2002263101 A